Europäisches Patentamt

European Patent Office

Office européen des brevets

(19)

(11) Veröffentlichungsnummer: **0 399 063 B1**

## EUROPÄISCHE PATENTSCHRIFT

(12)

(12)

(45) Veröffentlichungstag der Patentschrift: **05.01.94**

(21) Anmeldenummer: **89109172.0**

(22) Anmeldetag: **22.05.89**

(51) Int. Cl.⁵: **A61N 1/36**, A61N 1/08

(54) **Implantierbares medizinisches Gerät mit Mitteln zum Stimulieren von Gewebekontraktionen mit einstellbarer Stimulationsintensität und Verfahren zum Betrieb eines solchen Gerätes.**

(43) Veröffentlichungstag der Anmeldung:
**28.11.90 Patentblatt 90/48**

(45) Bekanntmachung des Hinweises auf die
Patenterteilung:
**05.01.94 Patentblatt 94/01**

(84) Benannte Vertragsstaaten:
**DE FR GB IT NL SE**

(56) Entgegenhaltungen:
**EP-A- 0 129 503**
**US-A- 4 773 401**

(73) Patentinhaber: **Siemens Elema AB**
**Röntgenvägen 2**
**S-171 95 Solna 1(SE)**

(84) Benannte Vertragsstaaten:
**SE**

(73) Patentinhaber: **SIEMENS AKTIENGESELL-**
**SCHAFT**
**Wittelsbacherplatz 2**
**D-80333 München(DE)**

(84) Benannte Vertragsstaaten:
**DE FR GB IT NL**

(72) Erfinder: **Högnelid, Kurt, Dipl.-Ing.**
**Järnvägsgatan 50**
**S-172 35 Sundbyberg(SE)**
Erfinder: **Norén, Kjell, Dipl.-Ing.**
**Karolinagatan 10C**
**S-171 58 Solna(SE)**
Erfinder: **Wecke Liliane, Dipl.-Ing.**
**Franstorpsvägen 4B**
**S-172 38 Sundbyberg(SE)**
Erfinder: **Engström, Jan, Dipl.-Ing.**
**Dagsverksvägen 213**
**S-163 55 Spanga(SE)**

EP 0 399 063 B1

# Beschreibung

Implantierbares medizinisches Gerät mit Mitteln zu Stimulieren von Gewebekontraktionen mit einstellbarer stimulationsintensität und Verfahren zum Betrieb eines solchen Gerätes

Die Erfindung betrifft ein in den Körper eines Lebewesens implantierbares medizinisches Gerät mit Mitteln zum elektrischen Stimulieren von Gewebekontraktionen mit einstellbarer Stimulationsintensität, mit einer Detektoreinrichtung zum Detektieren stimulierter Gewebekontraktionen und Stellmitteln zum Einstellen der Stimulationsintensität, die die Stimulationsintensität selbsttätig derart einstellen, dass die die Detektoreinrichtung nach einer Stimulation ein stimulierte Gewebekontraktionen detektiert. Die Erfindung betrifft ausserdem ein Verfahren zum Betrieb eines solchen Gerätes. Der Begriff Stimulationsintensität soll hier umfassend verstanden werden, d.h., dass die Dauer, die Häufigkeit, die Folgefrequenz, die Amplitude usw., mit der die Mittel zum Stimulieren tätig werden einzeln oder in Kombination ein Mass für die Stimulationsintensität darstellen können.

Bei derartigen Geräten erfolgt die selbsttätige Einstellung der Stimulationsintensität mit dem Ziel, einerseits sicherzustellen, dass jede Stimulation tatsächlich zu einer stimulierten Gewebekontraktion führt, und andererseits zu gewährleisten, dass dies nicht durch eine unnötig hohe Stimulationsintensität erreicht wird. Eine hohe Stimulationsintensität bedeutet in der Regel einen hohen Energiebedarf. Da im Falle implantierbarer medizinische Geräte als Energiequelle üblicherweise eine Batterie begrenzter Kapazität vorgesehen ist, ist eine unnötig hohe Stimulationsintensität im Interesse der Lebensdauer der Batterie und damit des Gerätes unter allen Umständen zu vermeiden.

Bei Geräten der eingangs genannten Art tritt das Problem auf, dass die Detektion einer stimulierten Gewebekontraktion in der Regel nur unmittelbar in Anschluss an eine Stimulation erfolgen kann. Zu diesem Zeitpunkt ist aber das mit der Stimulationsenergie beaufschlagte Gewebe infolge der Zufuhr elektrischer Energie noch polarisiert. Damit besteht die Gefahr, dass die Detektoreinrichtung übersteuert wird, so dass eine ordnungsgemässe Detektion nicht möglich ist. Es besteht zwar die Möglichkeit, die Detektoreinrichtung erst dann zu aktivieren, wenn bereits eine gewisser Potentialausgleich stattgefunden hat; jedoch besteht dann die Gefahr, dass zu diesem Zeitpunkt auch keine Detektion mehr möglich ist. Weiter besteht die Möglichkeit, unmittelbar im Anschluss an die Stimulation eine weitere Energiezufuhr vorzunehmen, die einen Potentialausgleich herbeiführt. Obwohl zum Potentialausgleich eine im Vergleich zur Stimulation geringe Energiemenge benötigt wird, ist diese zusätzliche Energiebedarf dennoch unerwünscht, da er sich jedenfalls negativ auf die Lebensdauer der Batterie auswirkt.

Aus der Druckschrift US-A-4 773 401 ist ein physiologisch gesteuerter Herzschrittmacher bekannt, bei welchem eine Impedanzmessung im rechten Ventrikel zur Erzeugung eines Kontrolparameters vorgenommen wird.

Der Erfindung liegt die Aufgabe zugrunde, ein Gerät der eingangs genannten Art so auszubilden, dass eine sichere Detektion stimulierte Ereignisse gewährleistet und ein energiesparender Betrieb des Gerätes sichergestellt ist. Der Erfindung liegt ausserdem der Aufgabe zugrunde, ein Verfahren zum Betrieb eines solchen Gerätes anzugeben, das einen zuverlässigen und energiesparenden Betrieb ermöglicht.

Nach der Erfindung wird der das Gerät betreffende Teil der Aufgabe dadurch gelöst, dass die Detektoreinrichtung stimulierte Gewebekontraktionen in einem der elektrischen Impedanz des stimulierten Gewebes entsprechenden Signal detektiert. Da die elektrische Impedanz des stimulierten Gewebes in ihrem zeitlichen Verlauf zwar der Kontraktionstätigkeit des Gewebes entspricht, jedoch in keiner Weise dadurch beeinflusst wird, dass das stimulierte Gewebe eventuell infolge der zugeführten Stimulationsenergie polarisiert ist, ist im Fall des erfindungsgemässen Gerätes eine sichere Detektion stimulierter Gewebekontraktionen möglich, ohne dass die Gefahr von Übersteuerungen der Detektoreinrichtung besteht oder eine Energiezufuhr zur Depolarisation des stimulierten Gewebes erforderlich ist. Allerdings sollte sichergestellt sein, dass dem der elektrischen Impedanz des stimulierten Gewebes entsprechenden Signal keine Gleichspannungsanteile überlagert sind.

Dies ist bei einer bevorzugten Ausführungsform der Erfindung gewährleistet, die im Patentanspruch 2 angegeben ist. Infolge des Umstandes, dass bei dieser Ausführungsform der Wechselspannungsanteil des über der modulierbaren Stromquelle abfallenden Spannung phasenrichtig demoduliert wird, wird ein Signal erhalten, das ausschliesslich den Impedanzverlauf des stimulierten Gewebes wiederspiegelt und das frei von durch eine Polarisation des Stimulierten Gewebes verursachten Störeinflüssen wie beispielsweise Gleichspannungsanteilen ist.

Im Falle der Ausführungsform der Erfindung gemäss Patentanspruch 3 liegt die eingestellte Stimulationsintensität jeweils um ein in Abhängigkeit von den bei dem jeweiligen Patienten vorliegenden individuellen Gegebenheiten wählbares Sicherheits-Marginal höher als diejenige Stimulationsintensität, die erforderlich ist, um sicherzustellen, dass jede Stimulation tatsächlich zu einer stimulierten Gewebekontraktion führt. Dabei entspricht der Mindestwert der Stimulationsintensität der sogenannten Reizschwelle, die diejenige Stimulationsintensität kennzeichnet, die wenigstens erforderlich ist, um eine stimulierte Gewebekontrations auszulösen. Da sich die Reizschwelle infolge der unterschiedlichsten Einflüsse (Hormonspiegel, Tageszeit etc.) ändert, sich die eingestellte Stimulationsintensität jedoch am zeitlichen Verlauf der Reizschwelle orientiert, ist unter allen Umständen ein sicherer Betrieb der erfindungsgemässen Gerätes gewährleistet, ohne dass die Stimulationsintensität und damit der Energiebedarf höher als erforderlich ist.

Obwohl Gewebekontraktionen nur dann stimuliert werden, wenn spontane Gewebekontraktionen ausbleiben, so dass die erforderliche Detektion spontaner Gewebekontraktionen grundsätzlich auch anhand des der elektrischen Impedanz entsprechenden Signals erfolgen kann, ist bei einer bevorzugten Ausführungsform der Erfindung gemäss Patentanspruch 5 vorgesehen, dass eine weitere Detektoreinrichtung zur Detektion spontaner Gewebekontraktionen vorgesehen ist, die diese anhand eines der Kontraktionstätigkeit des Gewebes entsprechenden elektrischen Signales detektiert. Dies bietet in Anbetracht des Umstandes, dass die Bildung eines der elektrischen Impedanz des stimulierten Gewebes entsprechenden Signals mit einem gewissen Energiebedarf verbunden ist, den Vorteil, dass die Detektoreinrichtung zur Detektion stimulierte Gewebekontraktionen nur für kurze Zeit unmittelbar im Anschluss an eine Stimulation aktiviert werden muss, so dass der Energiebedarf vernachlässigbar gering ist. Der Energiebedarf der weiteren zur Detektion spontaner Gewebekontraktionen dienenden Detektoreinrichtung ist deutlich geringer als der der Detektoreinrichtung für stimulierte Gewebekontraktionen.

Der das Verfahren betreffende Teil der Aufgabe ist erfindungsgemäss durch das im Patentanspruch 7 angegebene Verfahren gelöst. Demnach erfolgt also die Einstellung der Empfindlichkeit nicht kontinuierlich, sondern jeweils nur zu aufeinanderfolgenden ersten Zeitpunkten, wobei das Zeitintervall zwischen zwei aufeinanderfolgenden ersten Zeitpunkten beispielsweise eine Dauer in der Grössenordnung von Stunden besitzen kann. Die Detektoreinrichtung zur Detektion stimulierter Ereignisse braucht also nur in grösseren Zeitabständen in Betrieb genommen zu werden, was sich günstig auf den Energiebedarf des Gerätes und damit die Lebensdauer einer das Gerätspeisenden Batterie auswirkt. Da sprunghafte Änderungen der Reizschwelle in der Regel nicht auftreten, sind durch die beschriebene intermittierende Betriebsweise keine nachteiligen Folgen für den Patienten zu befürchten. Dennoch kann im Falle etwaiger Störungen rasch eine Korrektur der eingestellten Stimulationsintensität erfolgen, da gemäss Patentanspruch 8 zu zweiten Zeitpunkten, zwischen denen beispielsweise ein Zeitintervall mit einer Dauer in der Grössenordnung von Minuten liegt, überprüft wird, ob die eingestellte Stimulationsintensität noch hoch genug ist, um stimulierte Gewebekontraktionen auszulösen.

Die Verfahren gemäss den Patentansprüchen 9 bis 12 bieten den Vorteil, dass eine Stimulationsintensität eingestellt wird, die sich an dem Mindestwert der Stimulationsintensität orientiert, bei dem jede Stimulation eine stimulierte Muskelkontraktion auslöst. Hierdurch ist gewährleistet, dass die Stimulationsintensität nicht höher eingestellt wird, als dies, gegebenenfalls unter Berücksichtigung von Sicherheits-Marginalen, unbedingt erforderlich ist. Es ist also sichergestellt, dass einerseits die Stimulationen tatsächlich stimulierte Gewebekontraktionen hervorrufen und andererseits der Energiebedarf des Gerätes möglichst gering gehalten wird.

Die Verfahren gemäss den Patentansprüchen 13 bis 16 betreffen die Ermittlung des Mindestwertes der Stimulationsintensität. Dabei ist für den Fall eines Anstiegs der Reizschwelle sichergestellt, dass der entsprechende höhere Mindestwert der Stimulationsintensität rasch gefunden werden kann, so dass die Zahl von erfolglosen Stimulationen auf ein Mindestmass beschränkt ist. Für den Fall eines Absinkens der Reizschwelle wird der entsprechende geringere Mindestwert der Stimulationsintensität schrittweise zu mehreren ersten Zeitpunkten ermittelt. Dies bietet den Vorteil, dass bei einem nur kurzzeitigen Absinken der Reizschwelle die Stimulationsintensität nicht soweit gesenkt wird, dass die Gefahr besteht, dass Stimulationen erfolglos bleiben.

Mit dem Verfahren gemäss Patentanspruch 17 wird der Vorteil erzielt, dass während der selbsttätigen Einstellung der Stimulationsintensität wenigstens eine von zwei aufeinanderfolgenden Stimulationen eine stimulierte Gewebekontraktion auslöst, so dass auch während der selbsttätigen Einstellung der Stimulationsintensität eine ausreichende Stimulation des zu stimulierenden Gewebes sichergestellt ist.

Die Erfindung wird nachfolgend anhand der beigefügten Zeichnungen näher erläutert. Es zeigen:

Fig. 1 ein Blockschaltbild eines als Herzschrittmacher ausgebildeten erfindungsgemässen Gerätes,

Fig. 2 ein Blockschaltbild der zweiten Detektoreinrichtung des Herzschrittmachers gemäss Fig. 1,

Fig. 3 für eine Anzahl natürlicher Herzschläge das der elektrischen Aktivität des Herzens entsprechende Signal sowie das entsprechende der Impedanz des Herzens entsprechende Signal,

Fig. 4    ein ein Betriebsverfahren zur selbsttätigen Einstellung des Energiegehaltes der Stimulationsimpulse des Herzschrittmachers gemäss Fig. 1 verdeutlichendes Flussdiagramm, und

Fig. 5    ein ein Betriebsverfahren zur selbsttätigen Einstellung der Empfindlichkeit der ersten Detektoreinrichtung des Herzschrittmachers gemäss Fig. 1 verdeutlichendes Flussdiagramm.

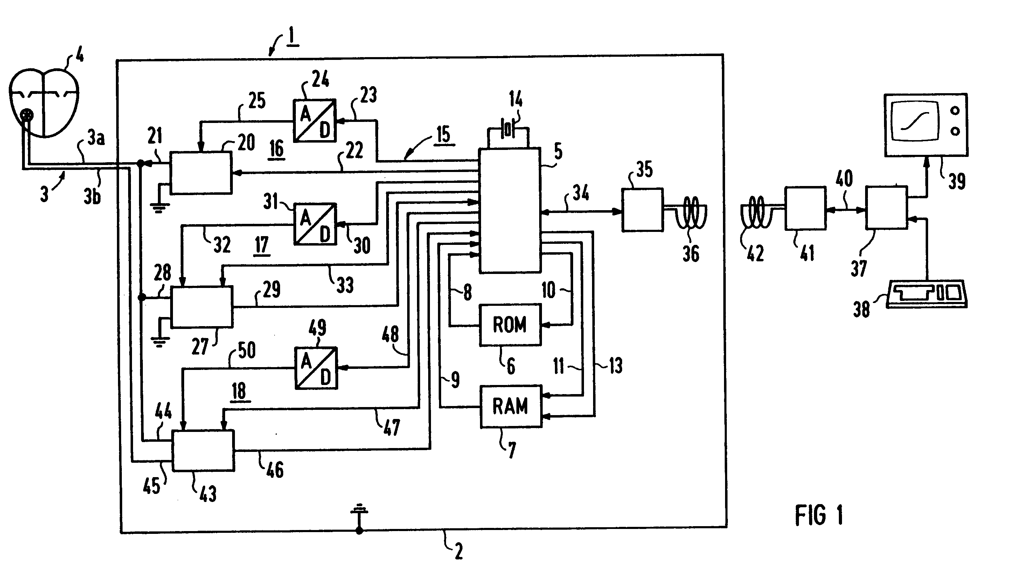

In der Fig. 1 ist die Erfindung anhand eines insgesamt mit (1) bezeichneten Herzschrittmachers dargestellt. Die Bauteile des Herzschrittmachers (1) sind in einem schematisch angedeuteten, hermetisch dichten Gehäuse aus einem elektrisch leitenden Werkstoff, z.B. Titan, aufgenommen. Von dem im VVI-Mode arbeitenden Herzschrittmacher (1) führt eine bipolare Elektrode (3) zu einem schematisch angedeuteten Herz (4) eines Lebewesens und ist dort in ein Ventrikel, vorzugsweise das rechte, eingepflanzt. Die bipolare Elektrode (3) besitzt zwei Leitungen (3a, 3b).

Der Herzschrittmacher (1) umfasst u.a. einen Mikroprozessor (5), dem ein Nur-Lese-Speicher (ROM) (6) und ein Schreib-Lese-Speicher (RAM) (7) zugeordnet sind, die über Datenleitungen (8, 9) und Adressleitungen (10, 11) mit dem Mikroprozessor (5) in Verbindung stehen. Zu dem RAM (7) führt von dem Mikroprozessor (5) ausserdem eine Leitung (13), die zum Umschalten des RAM (7) von Schreib-auf Lesebetrieb und umgekehrt dient. In dem ROM (6) ist ein Programm gespeichert, mittels dessen sämtliche Funktionen des Herzschrittmachers gesteuert werden. Wenn also im folgenden die Rede davon ist, dass der Mikroprozessor (5) eine bestimmte Funktion ausführt, so ist darunter zu verstehen, dass der Mikroprozessor (5) durch Ausführung des im ROM (6) gespeicherten Programms unter Heranziehung von in dem RAM (7) befindlichen Daten und ihm anderweitig, z.B. über eine Eingangs-Beschaltung, zugeführten Daten zur Ausführung der jeweiligen Funktion tätig wird. Wenn die Rede davon ist, dass der Mikroprozessor (5) für einen Parameter einen bestimmten Wert einstellt, so bedeutet dies in der Regel, falls nichts anderes angegeben ist, dass dem bestimmten Wert entsprechende Daten in dem RAM (7) gespeichert sind, auf die der Mikroprozessor (5) zugreifen kann.

Mit dem Mikroprozessor (5) ist ein Quarz (14) verbunden, der zur Erzeugung der für den Betrieb des Mikroprozessors (5) erforderlichen Taktsignale dient und ausserdem die Zeitreferenz für den Betrieb des Herzschrittmachers (1) darstellt.

Der Mikroprozessor (5) des Herzschrittmachers (1) besitzt eine insgesamt mit (15) bezeichnete Eingangs/Ausgangs-Beschaltung, die mehrere Kanäle (16, 17, 18) aufweist.

Der Kanal (16) dient dazu, das Herz (4) erforderlichenfalls mit Stimulationsimpulsen zu versorgen. Der Kanal (16) besitzt daher einen Stimulationsimpuls-Generator (20), der einen mittels einer Ausgangsleitung (21) mit der Leitung (3a) der Elektrode (3) verbundenen Signalausgang und einen ein Bezugspotential führenden Anschluss aufweist. Der das Bezugspotential führende Anschluss ist mit dem Gehäuse (2) elektrisch leitend verbunden. Dies ist dadurch veranschaulicht, dass sowohl der das Bezugspotential führende Anschluss des Stimulationsimpuls-Generators (20) als auch das Gehäuse (2) mit einem Massesymbol versehen sind. Obwohl eine bipolare Elektrode (3) vorhanden ist, erfolgt also die Stimulation unipolar. Der Stimulationsimpuls-Generator (20) ist über eine Leitung (22), die mit einem entsprechenden Ausgang des Mikroprozessors (5) verbunden ist, zur Abgabe eines Stimulationsimpulses aktivierbar. Digitale Daten, die die Dauer und die Amplitude und damit den Energiegehalt der Stimulationsimpulse betreffen, gelangen von dem Mikroprozessor (5) über eine Leitung (23) zu einer Digital/Analog-Schnittstelle (24), die dem Stimulationsimpuls-Generator (20) über eine Steuerleitung (25) den digitalen Daten entsprechende analoge Steuersignale zuführt. Diese stellen den Stimulationsimpuls-Generator (20) derart ein, dass er bei Bedarf Stimulationsimpulse eines bestimmten Energiegehaltes erzeugt und somit das Herz (4) des Lebewesens mit einer bestimmten Stimulationsintensität stimuliert.

Der Kanal (17) besitzt eine erste Detektoreinrichtung (27), die ausschliesslich dazu dient natürliche Herzschläge zu detektieren. Die erste Detektoreinrichtung (27) besitzt einen mit der Leitung (3a) der Elektrode (3) über eine Eingangsleitung (28) verbundenen Signaleingang und einen ein Bezugspotential führenden Anschluss. Dabei handelt sich bei dem Bezugspotential, wie durch das Massesymbol deutlich wird, um das gleiche Bezugspotential, das auch der entsprechende Anschluss des Stimulationsimpuls-Generators (20) und das Gehäuse (2) führen. Obwohl eine bipolare Elektrode (3) vorhanden ist, erfolgt also auch die Detektion natürlicher Herzschläge mittels der ersten Detektoreinrichtung (27) unipolar. Detektiert die zweite Detektoreinrichtung (27) in dem ihr über die Leitung (3a) der Elektrode (3) zugeführten, der elektrischen Aktivität des Herzens (4) entsprechenden Signal einen natürlichen Herzschlag, gibt sie über eine Leitung (29) ein dies anzeigendes Signal an einen entsprechenden Eingang des Mikroprozessors (5). Diese Signal gibt die erste Detektoreinrichtung (27) dann ab, wenn in dem der elektrischen Aktivität des Herzens (4) entsprechenden Signal ein Ereignis mit für einen natürlichen Herzschlag typischer Steilheit und/oder Amplitude auftritt. Der Mikroprozessor (5) ist über eine Leitung (30) mit einer Digital/Analog-Schnittstelle (31) verbunden, die ihr von dem Mikroprozessor (5) zugeführte digitale Daten als entsprechende analoge Signale über eine Steuerleitung (32) an die erste Detektoreinrichtung (27) weitergibt. Die digitalen Daten bzw. die entsprechenden analogen Signale dienen dazu, die Empfindlichkeit der zweiten Detektoreinrichtung (27), also diejenige Steilheit und/oder Amplitude einzustellen, die ein Ereignis in dem der elektrischen Aktivität des Herzens (4) entsprechenden Signal wenigstens aufweisen muss, um als natürlicher Herzschlag detektiert zu werden. Über eine Steuerleitung (33) kann der Mikroprozessor ausserdem der ersten Detektoreinrichtung (27) ein Signal zuführen, das diese völlig sperrt, so dass keine auf die Detektion eines natürlichen Herzschlages hinweisenden Signale zu dem Mikroprozessor (5) gelangen können.

Erhält der Mikroprozessor (5) über die Leitung (29) ein der Detektion eines natürlichen Herzschlages mittels der ersten Detektoreinrichtung (27) entsprechendes Signal oder aktiviert er über die Leitung (22) den Stimulationsimpuls-Generator (20) zur Abgabe eines Stimulationsimpulses, beginnt der Mikroprozessor (5) als Zähler zu arbeiten und beginnt eine Anzahl von aus der Schwingung des Quarzes (14) abgeleiteten Taktimpulsen abzuzählen, die einem Basis-Zeitintervall entspricht. Das Basis-Zeitintervall bestimmt diejenige Stimulationsfrequenz, mit der das Herz (4) beim Ausbleiben natürlicher Herzschläge stimuliert wird. Gelangt während des Basis-Zeitintervalls über den Kanal (17) kein Signal zu dem Mikroprozessor (5), das die Detektion eines natürlichen Herzschlags anzeigt, aktiviert der Mikroprozessor (5) bei Ablauf des Basis-Zeitintervalls über die Leitung (22) den Stimulationsimpuls-Generator (20). Im Anschluss an die Abgabe eines Stimulationsimpulses beginnt der Mikroprozessor (5) erneut eine Anzahl von Taktimpulsen abzuzählen, die dem die Stimulationsfrequenz bestimmenden Basis-Zeitintervall entspricht. Erhält der Mikroprozessor (5) dagegen während des Laufs des Basis-Zeitintervalls ein die Detektion eines natürlichen Herzschlags anzeigendes Signal von der ersten Detektoreinrichtung (27), bricht er den beschriebenen Zählvorgang ab, sofern ein weiteres Zeitintervall, die sogenannte Refraktärzeit, abgelaufen ist, und beginnt den beschriebenen Zählvorgang von neuem ohne dass die Abgabe eines Stimulationsimpulses erfolgt. Die Detektion des natürlichen Herzschlages mittels der ersten Detektoreinrichtung (27) inhibiert also die Abgabe eines Stimulationsimpulses. Die Refraktärzeit, die grundsätzlich kürzer ist als das beispielsweise auf Werte zwischen 400 und 2000 Millisekunden programmierbare Basis-Zeitintervall, dauert etwa zwischen 250 und 450 Millisekunden (programmierbar). Die Refraktärzeit unterteilt sich in eine absolute Refraktärzeit mit einer Dauer von gewöhnlich 125 Millisekunden und eine relative Refraktärzeit, auf die die restliche Zeit der gesamten eingestellten Refraktärzeit entfällt. Die Refraktärzeit beginnt jeweils gleichzeitig mit dem Basis-Zeitintervall zu laufen und wird von dem Mikroprozessor (5) im Zuge des gleichen Zählvorgangs ermittelt, der auch zur Ermittlung des Basis-Zeitintervalls dient. Während der absoluten Refraktärzeit ist im Kanal (17) die erste Detektoreinrichtung (27) gesperrt, was dadurch erreicht wird, das der Mikroprozessor (5) der ersten Detektoreinrichtung (27) über die Steuerleitung (33) ein entsprechendes Signal zuführt. Infolge der Sperrung der ersten Detektoreinrichtung (27) ist während der Dauer der absoluten Refraktärzeit mittels der ersten Detektoreinrichtung (27) keinerlei Detektion möglich, d.h. entsprechende Signale können nicht zu dem Mikroprozessor (5) gelangen. Nach Ablauf der absoluten Refraktärzeit aktiviert der Mikroprozessor (5) die erste Detektoreinrichtung (27) wieder, so dass diese in der Lage ist, natürliche Herzschläge zu detektieren. Erfolgt eine Detektion während der relativen Refraktärzeit, bricht der Mikroprozessor (5) im Gegensatz zu einer Detektion nach Ablauf der Refraktärzeit den Zählvorgang zur Ermittlung des Basis-Zeitintervalls nicht ab, sondern führt ihn fort und schliesst ihn mit einer Aktivierung des Stimulationsimpuls-Generators (20) ab. Allerdings setzt der Mikroprozessor (5) nach der Detektion eines natürlichen Herzschlages nochmals die volle Refraktärzeit in Gang. Hierdurch wird erreicht, dass im Falle von Hochfrequenzstörungen, die zu Fehldetektionen führen, unabhängig vom Auftreten natürlicher Herzschläge Stimulationsimpulse mit der durch das jeweils programmierte Basis-Zeitintervall bestimmten Stimulationsfrequenz erzeugt werden. Auch wenn die spontane Herzschlagfrequenz so hoch liegt, dass das Auftreten natürlicher Herzschläge jedesmal innerhalb der relativen Refraktärzeit erfolgt, erfolgt eine Abgabe von Stimulationsimpulsen mit der durch das jeweils programmierte Basis-Zeitintervall bestimmten Stimulationsfrequenz, und zwar bis die spontane Herzschlagfrequenz unter eine Frequenz zurückgefallen ist, deren Periodendauer der jeweils eingestellten Refraktärzeit entspricht. Mittels dieser Funktion ist die Beendigung bestimmter Wiedereintrittstachykardien möglich.

Der Mikroprozessor (5) ist über eine Leitung (34) mit einem Telemetrieschaltkreis (35) verbunden, an den eine Sende/Empfangs-Spule (36) angeschlossen ist. Der Herzschrittmacher (1) ist somit in der Lage, mit einem externen Progammiergerät (37) mit einer Tastatur (38) und einem Monitor (39) Daten auszutauschen, da das Programmiergerät (37) über eine Leitung (40) mit einem zweiten Telemetrieschaltkreis (41) verbunden ist, an den wieder eine Sende/Empfangs-Spule (42) angeschlossen ist. Zum Datenaustausch zwischen dem Herzschrittmacher (1) und dem Programmiergerät (37) wird die Sende/Empfangs-Spule (42) des zu dem Programmiergerät (37) gehörigen Telemetrieschaltkreises (41) so auf der Körperoberfläche des den Herzschrittmacher (1) tragenden Lebewesens positioniert, dass sie mit der Sende/Empfangs-Spule (36) des Herzschrittmachers (1) induktiv gekoppelt ist. Es besteht dann die Möglichkeit, dem Programmiergerät (27) die in dem ROM (6) und dem RAM (7) befindlichen Daten zur Überprüfung zuzuführen. Ausserdem besteht die Möglichkeit, dem RAM (7) des Herzschrittmachers (1) von dem Programmiergerät (37) her veränderte bzw. zusätzliche Daten zuzuführen, die das Betriebsverhalten des Herzschrittmachers (1), also dessen Zussamenspiel mit dem zu stimulierenden Herz (4) beeinflussen und verändern. Dieser Vorgang wird gewöhnlich als Programmierung bezeichnet.

Der Kanal (18) der Eingangs/Ausgangs-Beschaltung (15) des Mikroprozessors (5) dient dazu, dem Mikroprozessor (5) Daten zur Verfügung zu stellen, die es diesem anhand des in dem ROM (6) gespeicherten Programmes gestatten, einerseits die Stimulationsintensität, also den Energiegehalt der mittels des Stimulationsimpuls-Generator (20) erzeugten Stimulationsimpulse, so einzustellen dass ein Stimulationsimpuls auch tatsächlich einen stimulierten Herzschlag auslöst, und andererseits die Empfindlichkeit der ersten Detektoreinrichtung (27) so einzustellen, dass eine störungsfreie und sichere Detektion aller natürlicher Herzschläge gewährleistet ist. Der Kanal (18) enthält zu diesem Zweck eine zweite Detektoreinrichtung (43), die sowohl zur Detektion natürlicher als auch stimulierter Herzschläge vorgesehen ist. Die zweite Detektoreinrichtung (43) besitzt zwei Anschlüsse, von denen der eine über die Leitung (44) mit der Leitung (3a) und der andere über eine Leitung (45) mit der Leitung (3b) der bipolaren Elektrode (3) verbunden ist. Die zweite Detektoreinrichtung (43) detektiert im Gegensatz zu der bereits beschriebenen ersten Detektoreinrichtung (27) nicht nur natürliche, sondern wie bereits erwähnt auch stimulierte Herzschläge, also Herzschläge, die auf einen durch den Stimulationsimpuls-Generator (20) abgegebenen Stimulationsimpuls hin auftreten. Detektiert die zweite Detektoreinrichtung (43) einen stimulierten oder einen natürlichen, d.h. spontan auftretenden, Herzschlag, gelangt ein entsprechendes Signal über eine Leitung (46) vom Ausgang der zweiten Detektoreinrichtung (43) zu einem entsprechenden Eingang des Mikroprozessors (5). Der Mikroprozessor (5) ist in der Lage, über eine Steuerleitung (47) durch ein entsprechendes Signal den Ausgang der zweiten Detektoreinrichtung (43) zu sperren. Die Empfindlichkeit der zweiten Detektoreinrichtung (43) ist mittels des Mikroprozessors (5) einstellbar, indem dieser über die Leitung (48) einer Digital/Analog-Schnittstelle (49) digitale Daten zuführt, die die Digital/Analog-Schnittstelle (49) in ein entsprechendes analoges Signal umsetzt, das der zweiten Detektoreinrichtung (43) über eine Steuerleitung (50) zugeführt ist. Das analoge Signal bestimmt in noch näher zu erläuternder Weise die Empfindlichkeit der zweiten Detektoreinrichtung, die der Mikroprozessor (5) normalerweise höher als die Empfindlichkeit der ersten Detektoreinrichtung (27), wenigstens jedoch gleich hoch einstellt.

Anders als im Falle der ersten Detektoreinrichtung (27), deren Ausgang der Mikroprozessor (5) nur nach Ablauf der absoluten Refraktärzeit freischaltet, müssen im Falle der zweiten Detektoreinrichtung (43) Detektionen auch während der absoluten Refraktärzeit erfolgen können, um die Detektion stimulierter Herzschläge zu ermöglichen. Der Mikroprozessor (5) schaltet also den Ausgang der zweiten Detektoreinrichtung (43) nicht nur nach Ablauf der absoluten Refraktärzeit bis zum Auftreten eines natürlichen Herzschlages oder der Abgabe eines Stimulationsimpulses zur Detektion natürlicher Herzschläge frei, sondern ausserdem einige Millisekunden nach der Abgabe eines Stimulationsimpulses für ein kurzes Zeitintervall, z.B. 100 Millisekunden, zur Detektion stimulierter Herzschläge frei. Dabei ist jedoch wesentlich, dass weder die Detektion eines stimulierten Herzschlages noch die Detektion eines natürlichen Herzschlages mittels der zweiten Detektoreinrichtung (43) die Abgabe eines Stimulationsimpulses inhibieren kann. Dies kann nur durch die Detektion eines natürlichen Herzschlages mittels der ersten Detektoreinrichtung (27) unter den zuvor beschriebenen Voraussetzungen erfolgen.

Die selbsttätige Einstellung des Energiegehaltes der mittels des Stimulationsimpuls-Generators (20) erzeugten Stimulationsimpulse erfolgt derart, dass der Mikroprozessor (5) nach Abgabe eines Stimulationsimpulses prüft, ob von dem dann freigeschalteten Ausgang der zweiten Detektoreinrichtung (43) über die Leitung (46) ein die Detektion eines stimulierten Herzschlages anzeigendes Signal ankommt. Ist dies nicht der Fall, erhöht der Mikroprozessor (5) den Energiegehalt des nächsten Stimulationsimpulses, indem er der Digital/Analog-Schnittstelle (24) digitale Daten zuführt, die diese in ein analoges Signal umsetzt, das den Stimulationsimpuls-Generator (20) derart einstellt, dass dieser einen Stimulationsimpuls mit einem gegenüber dem zuvor vorhandenen Energiegehalt erhöhten Energiegehalt abgibt. Dies erfolgt solange, bis eine Einstellung für den Energiegehalt der Stimulationsimpulse gefunden ist, bei der die zweite Detektoreinrichtung (43) nach einem Stimulationsimpuls jeweils einen stimulierten Herzschlag detektiert. Dabei stellt der Mikroprozessor (5) über die Digital/Analog-Schnittstelle (24) den Stimulationsimpuls-Generator (20) derart ein, dass der Energiegehalt der erzeugten Stimulationsimpulse der Summe aus einem Mindest-Energiegehalt (Mindestwert des Energiegehaltes), bei dem die zweite Detektoreinrichtung (43) gerade noch nach der Abgabe eines Stimulationsimpulses einen stimulierten Herzschlag detektiert, und einem Sicherheitsmarginal, z.B. 50 % des Mindest-Energiegehaltes, entspricht. Dabei entspricht der Mindest-Energiegehalt der sogenannten Reizschwelle, die der Energiegehalt eines Stimulationsimpulses wenigstens erreichen muss, um einen stimulierten Herzschlag auslösen zu können.

Zur Ermittlung des Mindest-Energiegehaltes senkt der Mikroprozessor (5) den Energiegehalt der Stimulationsimpulse ausgehend von einem Wert, bei dem die zweite Detektoreinrichtung (43) nach jedem Stimulationsimpuls einer Folge von Stimulationsimpulsen einen stimulierten Herzschlag detektiert, allmählich so weit ab, bis wenigstens nach einzelnen Stimulationsimpulsen mittels der zweiten Detektoreinrichtung (43) kein stimulierter Herzschlag mehr detektierbar ist. Ausgehend von dem so gefundenen Wert des Energiegehaltes erhöht der Mikroprozessor (5) den Energiegehalt der Stimulationsimpulse allmählich wieder, und zwar gerade so weit, bis die zweite Detektoreinrichtung (42) nach jedem Stimulationsimpuls wieder einen stimulierten Herzschlag detektiert. Der so gefundene Wert stellt den Mindest-Energiegehalt der Stimulationsimpulse dar.

Durch die beschriebene Einstellung des Energiegehaltes der Stimulationsimpulse wird erreicht, dass einerseits die Sicherheit des Patienten gewährleistet ist, da die Stimulation stets mit Stimulationsimpulsen erfolgt, deren Energiegehalt um ein Sicherheitsmarginal oberhalb des Mindest-Energiegehaltes liegt. Andererseit ist sichergestellt, dass der Energiebedarf des Gerätes durch die Abgabe der Stimulationsimpulse nicht höher als nötig ist, da sich der Energiegehalt der Stimulationsimpulse stets an dem erforderlichen Mindest-Energiegehalts orientiert.

Die selbsttätige Einstellung der Empfindlichkeit der ersten Detektoreinrichtung (27) erfolgt derart, dass der Mikroprozessor (5) nach Ablauf der absoluten Refraktärzeit prüft, ob ein mittels der zweiten Detektoreinrichtung (43) detektierter natürlichen Herzschlag auch mittels der ersten Detektoreinrichtung (27) detektiert wurde. Dies ist möglich, da der Mikroprozessor (5) jeweils nach dem Ablauf der absoluten Refraktärzeit die erste und die zweite Detektoreinrichtung (27 bzw. 43) aktiviert. Erfolgt die Detektion eines natürlichen Herzschlages nur mittels der zweiten Detektoreinrichtung (43) erhöht der Mikroprozessor (5) die Empfindlichkeit der ersten Detektoreinrichtung (27), indem er der Digital/Analog-Schnittstelle (31) digitale Daten zuführt, die diese in ein analoges Signal umwandelt, das die Empfindlichkeit der ersten Detektoreinrichtung (27) derart einstellt, dass diese gegenüber dem zuvor eingestellten Wert erhöht ist. Dies erfolgt solange, bis eine Einstellung für die Empfindlichkeit der ersten Detektoreinrichtung (27) gefunden ist, bei der die erste Detektoreinrichtung (27) mittels der zweiten Detektoreinrichtung (43) detektierte natürliche Herzschläge ebenfalls detektiert. Es versteht sich, dass für diesen Vorgang die Empfindlichkeit der zweiten Detektoreinrichtung (43) wenigstens der jeweils eingestellten Empfindlichkeit der ersten Detektoreinrichtung (27) entsprechen muss. Zweckmässigerweise stellt jedoch der Mikroprozessor (5) mittels der Digital/Analog-Schnittstelle (49) die Empfindlichkeit der zweiten Detektoreinrichtung (43) deutlich höher als die Empfindlichkeit der ersten Detektoreinrichtung (27) ein. Dabei ist jedoch zu beachten, dass die Empfindlichkeit der zweiten Detektoreinrichtung (43) nicht beliebig erhöht werden darf, da sichergestellt sein muss, dass Störungen und Rauschen nicht zu Fehldetektionen führen. Dabei stellt der Mikroprozessor (5) über die Digital/Analog-Schnittstelle (31) die Empfindlichkeit der ersten Detektoreinrichtung (27) derart ein, dass sie um ein Sicherheits-Marginal der Empfindlichkeit höher als eine Mindest-Empfindlichkeit (Mindestwert der Empfindlichkeit) ist, bei der die erste Detektoreinrichtung (27) gerade noch einen mittels der zweiten Detektoreinrichtung (43) detektierten natürlichen Herzschlag ebenfalls detektiert. Als Sicherheits-Marginal kommen z.B. 50 % der Mindest-Empfindlichkeit in Frage.

Zur Ermittlung der Mindest-Empfindlichkeit senkt der Mikroprozessor (5) die Empfindlichkeit der ersten Detektoreinrichtung (27) ausgehend von einem Wert, bei dem die erste Detektoreinrichtung (27) jeden mittels der zweiten Detektoreinrichtung (43) detektierten natürlichen Herzschlag ebenfalls detektiert, allmählich so weit ab, bis wenigstens einzelne mittels der zweiten Detektoreinrichtung (43) detektierte natürliche Herzschläge mittels der ersten Detektoreinrichtung (27) nicht mehr detektierbar sind. Ausgehend von dem so gefundenen Wert der Empfindlichkeit erhöht der Mikroprozessor (5) die Empfindlichkeit der ersten Detektoreinrichtung (27) allmählich wieder, und zwar gerade so weit, bis die erste Detektoreinrichtung (27) jeden mittels der zweiten Detektoreinrichtung (43) detektierten natürlichen Herzschlag ebenfalls detektiert. Bei dem so gefundenen Wert der Empfindlichkeit der ersten Detektoreinrichtung (27) handelt es sich um die Mindest-Empfindlichkeit.

Die beschriebene Einstellung der Empfindlichkeit der ersten Detektoreinrichtung (27) bietet den Vorteil, dass einerseits im Interesse des Patienten durch das Sicherheits-Marginal der Empfindlichkeit gewährleistet ist, dass alle natürlichen Herzschläge mittels der ersten Detektoreinrichtung (27) tatsächlich detektiert werden können. Anderseits ist infolge des Umstandes, dass die Einstellung der Empfindlichkeit auf Grundlage der jeweiligen Mindest-Empfindlichkeit erfolgt, sichergestellt, dass die tatsächlich vorhandene Empfindlichkeit zu keinem Zeitpunkt höher ist, als dies im Interesse des Patienten unbedingt erforderlich ist, so dass die Gefahr von Fehldetektionen nur sehr gering ist.

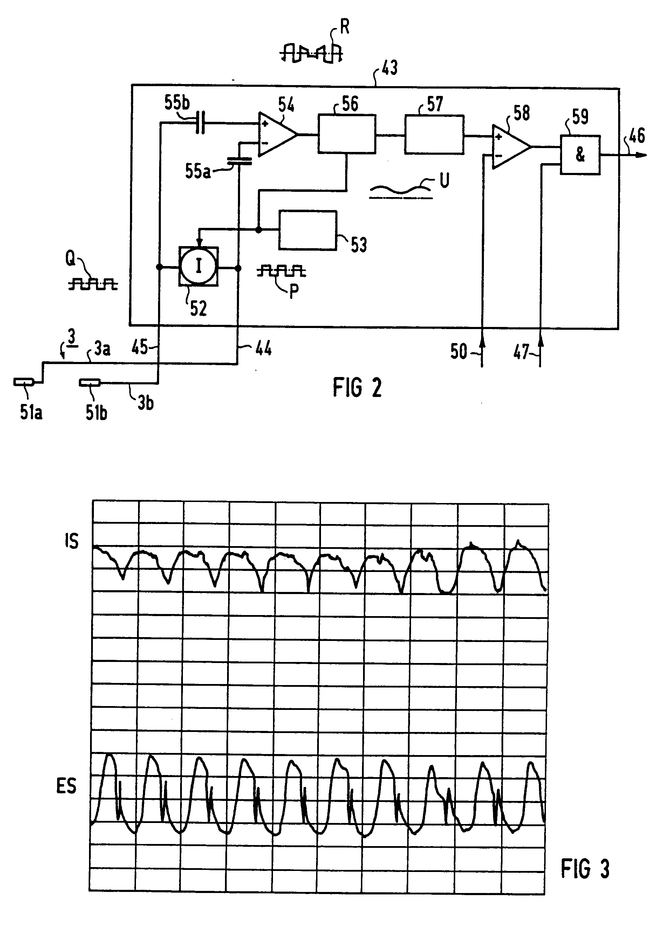

Ein Ausführungsbeispiel für die in Fig. 1 nur schematisch angedeutete zweite Detektoreinrichtung (43) ist in Fig. 2 dargestellt. Es handelt sich hier um eine Detektoreinrichtung, die ein Signal bildet, das der zwischen ihren Anschlüssen vorhandenen äusseren Impedanz entspricht. Dabei soll der Begriff äussere Impedanz zum Ausdruck bringen, dass in der äusseren Impedanz die Eingangsimpedanz der zweiten Detektoreinrichtung (43) nicht enthalten ist. Bei der äusseren Impedanz handelt es sich also sozusagen um die Quellimpedanz, die die zweite Detektoreinrichtung (43) sieht. Diese Quellimpedanz ergibt sich aus der Impedanz der in Fig. 2 schematisch angedeuteten bipolaren Elektrode (3) mit ihren Leitungen (3a und 3b) und der Impedanz des zwischen den bei implantierte Elektrode mit dem Herz in elektrisch leitender Verbindung stehenden Kontaktteilen (51a und 51b) befindlichen Herzmuskelgewebes.

Infolge der Herzaktion ändert sich die Impedanz des Herzmuskelgewebes, und zwar im Einklang mit der Herzaktion. Dies ist aus der Fig. 3 ersichtlich, in der für eine Anzahl natürlicher Herzschläge das der elektrischen Aktivität des Herzens entsprechende elektrische Signal, das mit ES bezeichnet ist, im Vergleich zu einem der elektrischen Impedanz der Herzens entsprechenden Signal, das mit IS bezeichnet ist, dargestellt ist.

Das der Impedanz des Herzens und damit der Herzaktion entsprechende elektrische Signal wird im Falle der zweiten Detektoreinrichtung (43) dadurch erhalten, dass diese eine bipolare modulierbare Stromquelle (52) aufweist, die parallell zu der Quellimpedanz zwischen die Anschlüsse der zweiten Detektoreinrichtung (43) geschaltet ist. Die Stromquelle (52) ist mit einem Oszillator (53) verbunden, der ein bei P angedeutetes Rechtecksignal mit einer konstanten Amplitude und mit einer konstanten Frequenz erzeugt, die deutlich grösser als die zu erwartender Herzschlagfrequenz ist. Als Frequenz des Rechtecksignals kommen z.B. 4 kHz infrage.

Der Oszillator (53) moduliert die Stromquelle (52) derart, dass diese einen Wechselstrom konstanter Amplitude erzeugt, der hinsichtlich seiner Frequenz und Kurvenform dem Signal des Oszillators (53) entspricht und zur Nullinie symmetrisch ist. Dieser Wechselstrom, der bei Q schematisch angedeutet ist, fliesst durch die an die zweite Detektoreinrichtung (43) angeschlossene Quellimpedanz.

Der über der Quellimpedanz infolge des Wechselstroms entstehende Spannungsabfall wird mittels eines Differenzverstärkers (54) verstärkt, dessen Eingänge über Koppelkondensatoren (55a und 55b) mit den Leitungen (45 und 46) verbunden sind. Infolge der Koppelkondensatoren (55a, 55b) verstärkt der Differenzverstärker (54) nur den durch den Wechselstrom hervorgerufenen Spannungsabfall, der ein Mass für die jeweils vorhandene Quellimpedanz darstellt. Da sich die Impedanz der Elektrode (3) nicht ändert, erfolgt die Änderung der Quellimpedanz ausschliesslich auf Grund der Impedanzänderungen des Herzens infolge der Herzaktion. Das Ausgangssignal des Differenzverstärkers (54), das bei R angedeutet ist und ebenfalls symmetrisch zur Nullinie verläuft, spiegelt also den Impedanzverlauf des Herzens und damit die Herzaktion wieder.

Das Ausgangssignal des Differenzverstärkers (54) ist einem Demodulator (56) zugeführt. Dieser demoduliert das Ausgangssignal des Differenzverstärkers (54) infolge des Umstandes, dass ihm das Signal des Oszillators (53) zugeführt ist, in korrekter Phasenbeziehung zu der Modulation der Stromquelle (52), so dass am Ausgang des Demodulators (56) ein bei U angedeutetes, in seinem Verlauf dem Impedanzverlauf des Herzens bzw. der Herzaktion entsprechendes unipolares Signal vorliegt, das im wesentlichen dem Signal IS gemäss Fig. 3 entspricht.

Dieses Signal ist einem Bandpassfilter (57) zugeführt, dessen Übertragungsfunktion derart gewählt ist, das nur diejenigen Signalanteile, die hinsichtliche ihr Frequenz bzw. Steilheit für Herzschläge, seien es natürliche oder stimulierte, typisch sind, das Bandpassfilter (57) passieren können.

Das Ausgangssignal des Bandpassfilters (57) gelangt zu dem einen Eingang eines Komparators (58), der dessen Amplitude mit einem Schwellwertsignals vergleicht, das seinem anderen Eingang über die Steuerleitung (50) zugeführt ist. Bei dem Schwellwertsignal handelt es sich um das Ausgangssignal der Digital/Analog-Schnittstelle (49). Überschreitet die Amplitude des Ausgangssignals des Bandpassfilters (57) den Pegel des Schwellwertsignals, springt das Ausgangssignal des Komparators (58) von seinem einen Extremwert auf seinen anderen Extremwert um, wobei bezüglich des Ausgangssignals des Komparators (58) der Ursprungzustand wieder hergestellt wird, wenn die Amplitude des Ausgangssignals des Bandpassfilters (57) unter das Schwellwertsignal fällt. Das Ausgangssignals des Komparators (58) besitzt den Wert logisch "0", solange die Amplitude des Ausgangssignals des Bandpassfilters (57) das Schwellwertsignal nicht überschreitet. Tritt eine Überschreitung des Schwellwertsignals auf, springt das Ausgangssignal des Komparators (58) auf logisch "1" um. Wenn also der Mikroprozessor (5) über die Digital/Analog-Schnittstelle (49) das Schwellwertsignals derart einstellt, dass es einem Pegel entspricht, die das bei natürlichen wie auch stimulierten Herzschlägen auftretende Ausgangssignal des Bandpassfilter (57) überschreitet, steht am Ausgang des Komparators (58) ein Signal zur Verfügung, das im Falle der Detektion eines Herzschlages den Pegel logisch "1" aufweist.

Das Ausgangssignal des Komparators (58) wird jedoch dem Mikroprozessor (5) nicht direkt zugeführt, sondern gelangt zu dem einen Eingang eines UND-Gliedes (59), dessen Ausgang mit der zu dem Mikroprozessor (5) führenden Leitung (46) verbunden ist. Mit dem anderen Eingang des UND-Gliedes (59) ist die Steuerleitung (47) verbunden. Es wird somit deutlich, dass nur dann die Detektion eines natürlichen oder stimulierten Herzschlages anzeigende Signale von dem Komparator (58) über die Leitung (46) zu dem Mikroprozessor (5) gelangen können, wenn dieser den Ausgang der zweiten Detektoreinrichtung (43) freischaltet, indem er dem UND-Glied (59) über die Steuerleitung (47) ein Signal mit dem Pegel logisch "1" zuführt, was nur während der im Zusammenhang mit der Fig. 1 erläuterten Zeitintervalle der Fall ist.

Es besteht ausserdem in in Fig. 2 nicht dargestellter Weise die Möglichkeit, die gesamte Detektoreinrichtung (43) während solcher Zeiträume, in denen sie nicht benötigt wird, stromlos zu schalten, um den Strombedarf des Herzschrittmachers zu senken.

Der Oszillator (53) muss nicht unbedingt als besondere Schaltung vorhanden sein. Das zur Modulation der Stromquelle (52) und zur Synchronisation des Demodulators (56) benötigte Rechtecksignal kann auch aus der Schwingung des mit dem Mirkoprozessor (5) verbundenen Quarzes (14) abgeleitet werden.

Die beschriebene Ausbildung der zweiten Detektoreinrichtung (43) bietet im Zusammenhang mit der Detektion stimulierter Herzschläge den Vorteil, dass das der Impedanz des Herzens entsprechende elektrische Signal ausschliesslich auf Grundlage desjenigen Spannungsabfalls über der Quellimpedanz ermittelt wird, der infolge des von der Stromquelle (52) abgegebenen Wechselstromes auftritt. Nachteilige Auswirkungen infolge des Umstandes, das unmittelbar nach einem Stimulationsimpuls, und zu dieser Zeit muss die Detektion eines stimulierten Herzschlages erfolgen, das Herzmuskelgewebe im Bereich des Kontaktes (51a) der Elektrode (3) ein von dem Potential des Herzmuskelgewebes im Bereich des Kontaktes (51b) abweichendes Potential aufweist, können also nicht auftreten.

Im Zusammenhang mit der selbsttätigen Einstellung der Empfindlichkeit der ersten Detektoreinrichtung (27) bietet die beschriebene Ausführung der zweiten Detektoreinrichtung (43) den Vorteil, dass die Detektion ein und desselben natürlichen Herzschlages mittels der ersten und der zweiten Detektoreinrichtung (27 bzw. 43) nicht gleichzeitig erfolgt, was bei der Verarbeitung der entsprechenden Signale mittels des Mikroprozessors (5) zu Problemen führen könnte. Vielmehr erfolgt die Detektion mittels der zweiten Detektoreinrichtung (43) kurze Zeit nach der entsprechenden Detektion mittels der ersten Detektoreinrichtung (27). Im Zusammenhang mit der selbsttätigen Einstellung der Empfindlichkeit der ersten Detektoreinrichtung (27) muss der Mikroprozessor (5) also nur überprüfen, ob der Detektion eines natürlichen Herzschlages mittels der zweiten Detektoreinrichtung (43) eine entsprechende Detektion mittels der ersten Detektoreinrichtung (27) vorangegangen ist. Störungen der ersten Detektoreinrichtung (27) durch den Wechselstrom der zweiten Detektoreinrichtung (43) sind nicht zu befürchten, da die Frequenz des Wechselstromes so hoch liegt, dass die erste Detektoreinrichtung darauf nicht ansprechen kann.

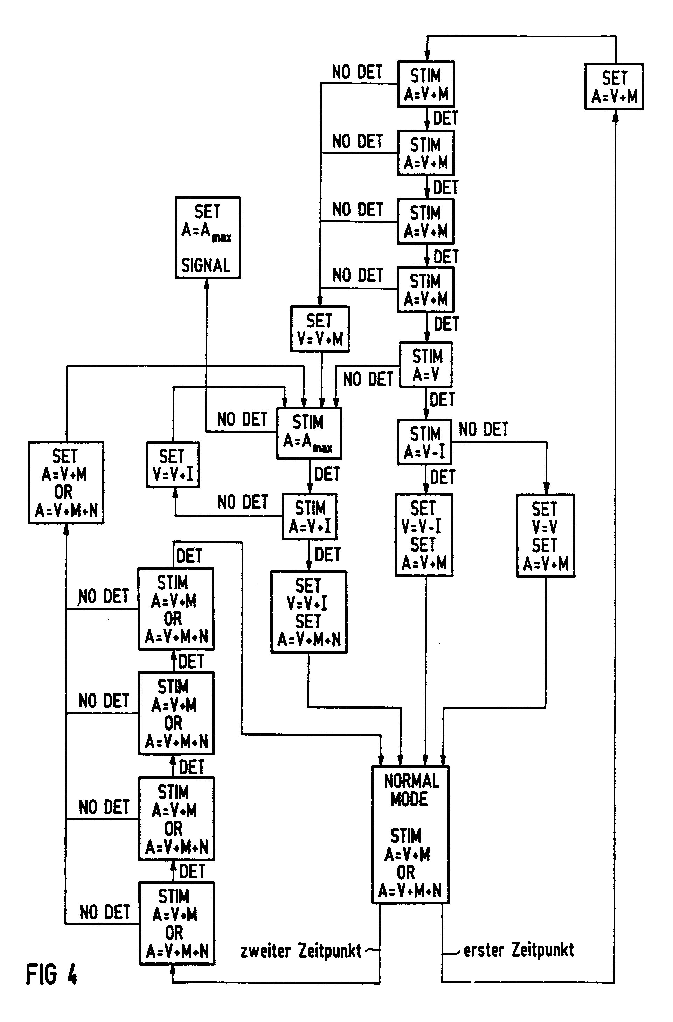

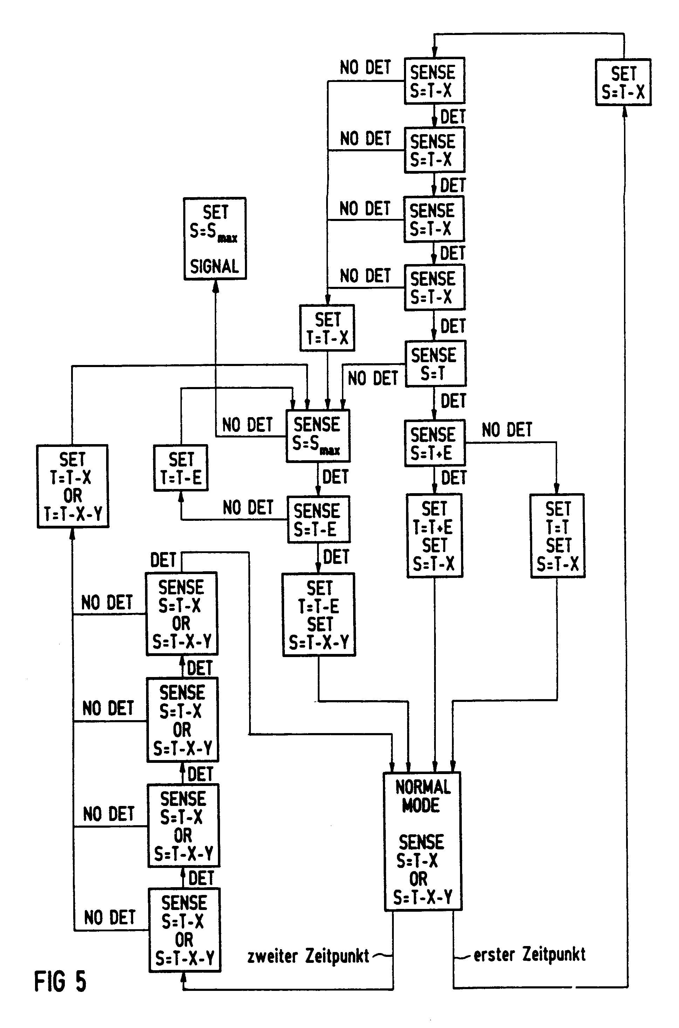

Nachdem im Zusammenhang mit der Fig. 1 bereits beschrieben wurde, wie die selbsttätige Einstellung der Stimulationsintensität und die Einstellung der Empfindlichkeit erfolgen können, werden diesbezügliche Betriebsverfahren im Zusammenhang mit den Figuren 4 und 5 näher erläutert.

Die Fig. 4 verdeutlicht das Betriebsverfahren zur selbsttätigen Einstellung der Stimulationsintensität, also des Energiegehaltes der Stimulationsimpulse. Demnach findet keine kontinuierliche Einstellung der Stimulationsintensität statt. Vielmehr befindet sich der Herzschrittmacher die meiste Zeit in einer mit "NORMAL MODE" bezeichneten Betriebsweise, in der die zweite Detektoreinrichtung (43) stromlos geschaltet ist und der Mikroprozessor (5) in keiner Weise zur Einstellung des Energiegehalts der Stimulationsimpulse tätig wird. Dies geschieht aus Gründen der Energieersparnis und ist ohne Nachteil für den Patienten möglich, da sich die Reizschwelle in der Regel nur sehr langsam ändert, so dass es genügt, eine selbsttätige Einstellung des Energiegehaltes der Stimulationsimpulse in grösseren Zeitabständen vorzunehmen. Im vorliegenden Falle erfolgt dies zu ersten Zeitpunkten, zwischen denen jeweils ein Zeitintervall in der Grössenordnung von Stunden liegt. Während dieser Zeitintervalle stimuliert der Stimulationsimpuls-Generator (20) das Herz mit Stimulationsimpulsen desjenigen Energiegehaltes A, der bei der zuletzt vorgenommenen selbsttätigen Einstellung des Energiegehaltes eingestellt wurde. Dieser Energiegehalt A setzt sich zusammen aus dem der Reizschwelle entsprechenden Mindest- Energiegehalt V, der erforderlich ist, um einen mittels der zweiten Detektoreinrichtung (43) detektierbaren stimulierten Herzschlag auszulösen, und dem mit M bezeichneten Sicherheits-Marginal. In besonderen Fällen, die noch erläutert werden, kann noch ein zusätzliches Sicherheits-Marginal N, das z.B. 25 % des Mindest-Energiegehalts V betragen kann, vorhanden sein.

Dass der Stimulationsimpuls-Generator (20) im "NORMAL MODE" mit dem erwähnten Energiegehalt (A) stimuliert, ist durch die Angabe STIM A = V + M bzw. STIM A = V + M + N verdeutlicht. In der Fig. 4 bedeutet die Angabe STIM verbunden mit der Angabe eines bestimmten Energiegehaltes A übrigens immer, dass ein Stimulationsimpuls des jeweiligen Energiegehaltes A abgegeben wird.

Im Zuge der selbsttätigen Einstellung der Stimulationsintensität, die zu jedem ersten Zeitpunkt erfolgt, wird die zweite Detektoreinrichtung aktiviert und zunächst für eine definierte Anzahl von Stimulationsimpulsen, in Fig. 4 sind es beispielhaft vier Stimulationsimpulse mit dem Energiegehalt A = V + M, überprüft, ob die zweite Detektoreinrichtung (43) jeweils einen stimulierten Herzschlag detektiert. Die Detektion eines stimulierten Herzschlages ist in Fig. 4 durch die Angabe DET veranschaulicht während das Ausbleiben der Detektion eines stimulierten Herzschlages nach einer Stimulation mit der Angabe NO DET verdeutlicht ist. Der Energiegehalt A der Stimulationsimpulse beträgt dabei übrigens unabhängig davon, ob zuvor das zusätzliches Sicherheits-Marginal N vorhanden war, A = V + M.

Wird für jeden der definierten Anzahl von Stimulationsimpulsen ein nachfolgender stimulierter Herzschlag detektiert, besitzt der nächste Stimulationsimpuls den zuletzt gefundenen Mindest-Energiegehalt V. Führt auch dieser Stimulationsimpuls zur Detektion eines stimulierten Herzschlages, wird bei der nachfolgenden Stimulation ein Stimulationsimpuls mit einem nochmals um einen definierten Schritt I verringerten Energiegehalt A = V-I abgegeben. Der Schritt I kann z.B. ein bestimmter Bruchteil des maximal möglichen Energiegehaltes $A_{max}$ der Stimulationsimpulse sein. Führt auch dieser Stimulationsimpuls zur Detektion eines stimulierten Herzschlages, bedeutet dies, dass die Reizschwelle abgesunken ist. Als neuer Mindest-Energiegehalt V wird daher der zuletzt vorhandene Wert vermindert um den Schritt I gesetzt und als neuer Energiegehalt A der Stimulationsimpulse der neue Mindest-Energiegehalt V erhöht um das Sicherheits-Marginal M gesetzt. Dies wird in Fig. 4 durch die Angaben SET V = V-I und SET A = V + M. Im übrigen bedeutet in Fig. 4 die Angabe SET in Verbindung mit der Angabe eines Parameters immer, dass diesem Parameter ein neuer Wert zugeordnet wird. Mit dem neu gesetzten Energiegehalt A, der wohlgemerkt um den Schritt I geringer ist, als der zuvor vorhandene, stimuliert der Herzschrittmacher dann im "NORMAL MODE".

Es wäre zwar grundsätzlich denkbar, dass die Reizschwelle zwischen zwei aufeinanderfolgenden ersten Zeitpunkten so weit absinkt, dass eine Absenkung des Mindest-Energiegehaltes V um mehr als einen Schritt I möglich wäre. Dennoch wird im Interesse der Sicherheit des Patienten nur um einen Schritt I abgesenkt. Sollte sich die Reizschwelle tatsächlich auf einem Niveau stabilisieren, dass eine weitere Absenkung des Energiegehaltes A gestattet, so erfolgt die weitere Absenkung im Zuge der nächsten selbsttätigen Einstellung(en) des Energiegehaltes A noch frühzeitig genug.

Führt der Stimulationsimpuls mit dem Energiegehalt A = V-I nicht zur Detektion eines stimulierten Herzschlages, bedeutet dies, dass sich die Reizschwelle seit dem letzten ersten Zeitpunkt nicht verändert hat. Daher werden wieder der alte Mindest-Energiegehalt V und der alte Energiegehalt A gesetzt, bevor die Rückkehr in den "NORMAL MODE" erfolgt.

Führt bereits die Stimulation mit dem zuletzt gefundenen Mindest-Energiegehalt V nicht zur Detektion eines stimulierten Herzschlages, ist also die Reizschwelle angestiegen, wird zunächst ein Stimulationsimpuls mit dem maximal möglichen Energiegehalt $A_{max}$ abgegeben, um sicherzustellen, dass nicht mehr als ein Stimulationsimpuls erfolglos bleibt.

Wird nach diesem Stimulationsimpuls mit dem maximalen Energiegehalt $A_{max}$ ein stimulierter Herzschlag detektiert, erfolgt als nächstes eine Stimulation mit einem Energiegehalt A = V + I, der dem letzten Mindest-Energiegehalt V erhöht um einen Schritt I entspricht. Führt dies zur Detektion eines stimulierten Herzschlages, wird der neue Mindest-Energiegehalt V entsprechend festgesetzt. Als neuer Energiegehalt A wird jedoch anders als zuvor nicht nur die Summe aus dem neuen Mindest-Energiegehalt V und dem Sicherheits-Marginal M eingestellt. Vielmehr erfolgt noch eine Erhöhung des Energiegehaltes A um das bereits erwähnte zusätzliche Sicherheits-Marginal N. Dadurch wird bis zum nächsten ersten Zeitpunkt, d.h. bis zur nächsten selbsttätigen Einstellung des Energiegehaltes A, im "NORMAL MODE" mit einem Energiegehalt A = V + M + N stimuliert. Dies geschieht, um für den Fall eines weiteren Ansteigens der Reizschwelle im Interesse der Sicherheit des Patienten bereits entsprechende Vorkehrungen zu treffen. Für den Fall, dass bis zum nächsten ersten Zeitpunkt kein weiterer Anstieg der Reizschwelle auftritt, entfällt, wie sich aus den vorstehenden Ausführungen ergibt, dass zusätzliche Sicherheits-Marginal N wieder.

Führt die Stimulation mit dem um einen Schritt I erhöhten Mindest-Energiegehalt V nicht zur Detektion eines natürlichen Herzschlages, wird der Mindest-Energiegehalt V um einen weiteren Schritt I erhöht. Vor einer Stimulation mit dem nochmals erhöhten Mindest-Energiegehalt V erfolgt aber zunächst eine Stimulation mit dem maximalen Energiegehalt $A_{max}$ Erst wenn danach ein stimulierter Herzschlag detektiert wird, erfolgt die Stimulation mit dem nochmals erhöhten Mindest-Energiegehalt V. Dies wiederholt sich solange, bis ein Mindest-Energiegehalt V gefunden ist, der zur Detektion eines stimulierten Herzschlages führt, worauf der neue Energiegehalt A für den "NORMAL MODE" wie unmittelbar vorstehend beschrieben eingestellt wird, der entsprechend der Anzahl von Schritten I und um das zusätzliche Sicherheits-Marginal N höher ist als der vorher vorhandene Energiegehalt A.

Führt auch eine Stimulation mit dem maximalen Energiegehalt $A_{max}$ nicht zur Detektion eines stimulierten Herzschlages, wird als Energiegehalt A für alle weiteren Stimulationen der maximale Energiegehalt $A_{max}$ eingestellt und die zweite Detektoreinrichtung (43) stromlos geschaltet. Ausserdem werden, dies ist durch die Angabe SIGNAL versinnbildlicht, Massnahmen getroffen, die bei der nächsten Kommunikation des Programmers (37) mit dem Herzschrittmacher (1) dazu führen, dass eine Meldung ausgegeben wird, durch die der behandelnde Arzt erkennen kann, dass eine selbsttätige Einstellung des Energiegehaltes A der Stimulationsimpulse nicht möglich war und deshalb auf den maximalen Energiegehalt $A_{max}$ übergegangen wurde.

Führt bereits einer der definierten Anzahl von Stimulationsimpulsen, die wie beschrieben zu Beginn der selbsttätigen Einstellung des Energiegehaltes A der Stimulationsimpulse abgegeben werden, nicht zur Detektion eines stimulierten Herzschlages, bedeutet dies, dass die Reizschwelle, z.B. durch eine Verlagerung des in das Herz eingepflanzten Endes der Elektrode, so weit angestiegen ist, dass ein Mindest-Energiegehalt V der Stimulationsimpulse erforderlich ist, der grösser ist als der Energiegehalt $A = V + M$. Es wird daher zunächst der Mindest-Energiegehalt V auf $V = V + M$ erhöht, bevor die Abgabe eines Stimulationsimpulses mit maximalem Energiegehalt $A_{max}$ erfolgt, an die sich die zuvor beschriebenen Verfahrensschritte zur Ermittlung eines neuen Mindest-Energiegehalts V und die Festsetzung des neuen Energiegehaltes A unter Berücksichtigung des zusätzlichen Sicherheits-Marginals N anschliessen, bevor die Rückkehr in den "NORMAL MODE" erfolgt.

Zwischen zwei aufeinanderfolgenden ersten Zeitpunkten wird wenigstens zu einem zweiten Zeitpunkt, sind mehrere zweite Zeitpunkte vorgesehen, sind diese jeweils durch ein Zeitintervall mit einer Dauer in der Grössenordnung von beispielsweise Minuten voneinander getrennt, geprüft, ob nach einer definierten Anzahl von Stimulationsimpulsen mit dem zuletzt eingestellten Energiegehalt, also $A = V + M$ oder $A = V + M + N$, jeweils die Detektion eines stimulierten Herzschlages erfolgt. Zu diesem Zweck wird der "NORMAL MODE" verlassen und die zweite Detektoreinrichtung (43) aktiviert. Wird für alle Stimulationsimpulse der definierten Anzahl, im Falle der Fig. 4 sind dies vier, das Auftreten eines stimulierten Herzschlages detektiert, erfolgt die Rückkehr in den "NORMAL MODE". Bleibt dagegen für einen der Stimulationsimpulse die Detektion eines stimulierten Herzschlages aus, erfolgt als nächstes eine Stimulation mit dem maximalen Energiegehalt $A_{max}$. Im Anschluss daran wird in der bereits beschriebenen Weise ein neuer Mindest-Energiegehalt V und ein neuer Energiegehalt $A = V + M + N$ der Stimulationsimpulse festgesetzt und mit diesem Wert in den "NORMAL MODE" zurückgekehrt.

Die beschriebene Kontrolle zu den zweiten Zeitpunkten erfolgt ebenfalls im Interesse der Sicherheit des Patienten, um etwaigen Störungen oder abnormalen Änderungen der Reizschwelle rasch Rechnung tragen zu können.

Wie ein Vergleich der Figuren 4 und 5 zeigt, ähnelt das in Fig. 5 dargestellte Betriebserfahren zur selbsttätigen Einstellung der Empfindlichkeit der ersten Detektoreinrichtung (27) dem Betriebsverfahren zur selbsttätigen Einstellung des Energiegehaltes der Stimulationsimpulse.

Auch bezüglich der Empfindlichkeit der ersten Detektoreinrichtung (27) findet also keine kontinuierliche Einstellung statt. Vielmehr befindet sich der Herzschrittmacher die meiste Zeit in seiner mit "NORMAL MODE" bezeichneten Betriebsweise, in der die zweite Detektoreinrichtung (43) stromlos geschaltet ist. Dies ist auch im Hinblick auf die Einstellung der Empfindlichkeit der ersten Detektoreinrichtung (27) ohne Nachteil für den Patienten möglich, da sich die Verhältnisse in der Regel nicht so rasch ändern, dass eine kontinuierliche Einstellung der Empfindlichkeit der ersten Detektoreinrichtung (27) erforderlich wäre. Es genügt also eine Einstellung der Empfindlichkeit zu ersten Zeitpunkten vorzunehmen, zwischen denen jeweils ein Zeitintervall mit einer Dauer in der Grössenordnung von Stunden liegt. Die ersten Zeitpunkte zur selbsttätigen Einstellung der Empfindlichkeit der ersten Detektoreinrichtung (27) sind zweckmässigerweise so gewählt, dass sie unmittelbar vor oder unmittelbar nach den ersten Zeitpunkten liegen, zu denen die selbsttätige Einstellung des Energiegehaltes der Stimulationsimpulse erfolgt.

Während der zwischen aufeinanderfolgenden ersten Zeitpunkten liegenden Zeitintervalle entspricht die Empfindlichkeit der ersten Detektoreinrichtung (27) der bei der zuletzt vorgenommenen selbsttätigen Einstellung eingestellten Empfindlichkeit. Diese Empfindlichkeit S setzt sich zusammen aus der Mindest-Empfindlichkeit T, die erforderlich ist, um einen mittels der zweiten Detektoreinrichtung (43) detektiereten natürlichen Herzschlag auch mit der ersten Detektoreinrichtung (27) detektieren zu können, und dem mit X bezeichneten Sicherheits-Marginal der Empfindlichkeit. In besonderen Fällen, die noch erläutert werden, kann noch ein zusätzliches Sicherheits-Marginal der Empfindlichkeit Y, das z.B. 25 % der Mindest-Empfindlichkeit T betragen kann, vorhanden sein.

Dass die erste Detektoreinrichtung (27) auf die erwähnte Empfindlichkeit S eingestellt ist, ist durch die Angabe SENSE S = T-X bzw. SENSE S = T-X-Y verdeutlicht. Dabei werden die Sicherheits-Marginale X bzw. Y von der Mindest-Empfindlichkeit T subtrahiert, da eine höhere Empfindlichkeit S in der Regel einem kleineren Zahlenwert der Empfindlichkeit S entspricht. In der Fig. 5 bedeutet die Angabe SENSE verbunden mit der Angabe einer bestimmten Empfindlichkeit S übrigens immer, dass die Empfindlichkeit S der ersten Detektoreinrichtung (27) auf den jeweiligen Wert eingestellt ist.

Im Zuge der selbsttätigen Einstellung der Empfindlichkeit S der ersten Detektoreinrichtung (27), die zu jedem ersten Zeitpunkt erfolgt, wird die zweite Detektoreinrichtung (43) aktiviert. Dann wird für eine definierte Anzahl von mittels der zweiten Detektoreinrichtung (43) detektierten natürlichen Herzschlägen, in Fig. 5 sind es beispielsweise vier natürliche Herzschläge, überprüft, ob diese auch mittels der ersten Detektoreinrichtung (27) detektiert werden, die auf die Empfindlichkeit S = T-X eingestellt ist, und zwar unabhängig davon, ob zuvor im "NORMAL MODE" auch das zusätzliche Sicherheits-Marginal Y vorhanden war. Die Detektion eines natürlichen Herzschlages mittels der ersten Detektoreinrichtung (27) ist in Fig. 5 durch die Angabe DET veranschaulicht. Bleibt die Detektion eines mittels der zweiten Detektoreinrichtung (43) detektierten Herzschlages durch die erste Detektoreinrichtung (27) aus, ist dies durch die Angabe NO DET verdeutlicht.

Werden alle natürlichen Herzschläge der definierten Anzahl von mittels der zweiten Detektoreinrichtung (43) detektierten natürlichen Herzschlägen auch mittels der ersten Detektoreinrichtung (27) detektiert, wird die Empfindlichkeit S der ersten Detektoreinrichtung (27) auf die zuletzt gefundene Mindest-Empfindlichkeit T eingestellt. Detektiert dann die erste Detektoreinrichtung (27) den nächsten mittels der zweiten Detektoreinrichtung (43) detektierten natürlichen Herzschlag, wird die Empfindlichkeit S der ersten Detektoreinrichtung (27) nochmals um einen definierten Schritt E auf S = T+E verringert. Der Schritt E kann z.B ein bestimmter Bruchteil oder ein Vielfaches der maximal möglichen Empfindlichkeit $S_{max}$ der ersten Detektoreinrichtung (27) sein. Detektiert die erste Detektoreinrichtung (27) mit der Empfindlichkeit S = T+E den nächsten mittels der zweiten Detektoreinrichtung (43) detektierten natürlichen Herzschlag ebenfalls, wird als neue Mindest-Empfindlichkeit T der ersten Detektoreinrichtung (27) T = T+E und als neue Empfindlichkeit S der ersten Detektoreinrichtung (27) S = T-X gesetzt, was durch die Angaben SET T = T+E und SET S = T-X verdeutlicht ist. Im übrigen bedeutet in der Fig. 5 die Angabe SET in Verbindung mit der Angabe eines Parameters immer, dass eine neue Festssetzung des entsprechenden Parameters auf den angegebenen Wert erfolgt. Auf die erwähnte neue Empfindlichkeit S, die wohlgemerkt um den Schritt E geringer ist, als die zuvor vorhandene Empfindlichkeit, wird die erste Detektoreinrichtung (27) dann im "NORMAL MODE" eingestellt.

Es wäre zwar möglich, dass sich die Verhältnisse zwischen zwei aufeinanderfolgenden ersten Zeitpunkten so stark ändern, dass eine Verringerung der Mindest-Empfindlichkeit T um mehr als einen Schritt E möglich wäre. Dennoch wird im Interesse der Sicherheit des Patienten eine Verringerung nur um einen Schritt E vorgenommen. Sollten sich die Verhältnisse tatsächlich auf einem Niveau stabilisieren, das eine weitere Verringerung der Empfindlichkeit S der zweiten Detektoreinrichtung (27) gestattet, so erfolgt diese im Zuge der nächsten selbsttätigen Einstellung(en) der Empfindlichkeit S.

Detektiert die erste Detektoreinrichtung (27) mit der Empfindlichkeit S = T+E den nächsten mittels der zweiten Detektoreinrichtung (43) detektierten natürlichen Herzschlag nicht, bedeutet dies, dass sich die Verhältnisse seit dem letzten ersten Zeitpunkt nicht verändert haben. Daher werden die Mindest-Empfindlichkeit T und die Empfindlichkeit S wieder auf die zuvor vorhandenen Werte eingestellt, bevor die Rückkehr in den "NORMAL MODE" erfolgt.

Detektiert die erste Detektoreinrichtung (27) bereits mit der zuletzt vorhandenen Mindest-Empfindlichkeit T einen mittels der zweiten Detektoreinrichtung (43) detektierten natürlichen Herzschlag nicht, bedeutet dies, dass eine Erhöhung der Mindest-Empfindlichkeit T erfolgen muss. Um die ordnungsgemässe Funktion des Herzschrittmachers im Interesse des Patienten möglichst weitgehend aufrechterhalten zu können, wird jedoch zunächst bis zur Detektion des nächsten natürlichen Herzschlages die Empfindlichkeit S auf ihren maximalen Wert $S_{max}$ eingestellt, der jedoch so gewählt ist, dass Störungen und Muskelzuckungen nicht zu Fehldetektionen führen können. Wird der nächste mittels der zweiten Detektoreinrichtung (43) detektierte natürliche Herzschlag auch mit der auf maximale Empfindlichkeit $S_{max}$ eingestellten ersten Detektoreinrichtung (27) detektiert, wird die erste Detektoreinrichtung (27) auf eine Empfindlichkeit S eingestellt, die der zuletzt vorhandenen, um den Schritt E erhöhten Mindest-Empfindlichkeit T entspricht. Detektiert die erste Detektoreinrichtung (27) mit dieser Empfindlichkeit S den nächsten mittels der zweiten Detektoreinrichtung (43) detektierten natürlichen Herzschlag, wird die neue Mindest-Empfindlichkeit T entsprechend festgesetzt. Als neue Empfindlichkeit S wird jedoch anders als zuvor nicht nur die um das Sicherheits-Marginal X erhöhte Mindest-Empfindlichkeit T eingestellt. Vielmehr erfolgt eine weitere Erhöhung um das bereits erwähnte zusätzliche Sicherheits-Marginal Y. Bis zum nächsten ersten Zeitpunkt ist also im "NORMAL MODE" die Empfindlichkeit der ersten Detektoreinrichtung (27) auf S = T-X-Y eingestellt. Dies geschieht, um für den Fall, dass eine weitere Erhöhung der Mindest-Empfindlichkeit T erforderlich werden sollte, bereits entsprechende Vorkehrungen im Interesse der Sicherheit des Patienten zu treffen. Für den Fall, dass bis zum nächsten ersten Zeitpunkt keine weitere Erhöhung der Mindest-Empfindlichkeit T erforderlich wird, entfällt, wie sich aus den vorstehenden Ausführungen ergibt, das zusätzliche Sicherheits-Marginal Y wieder.

Reicht die um einen Schritt E erhöhte Mindest-Empfindlichkeit T der ersten Detektoreinrichtung (27) nicht aus, um einen mittels der zweiten Detektoreinrichtung (43) detektierten natürlichen Herzschlag ebenfalls zu detektieren, wird die Mindest-Empfindlichkeit T um einen weiteren Schritt E erhöht. Zunächst wird aber die Empfindlichkeit der ersten Detektoreinrichtung (27) auf ihren maximalen Wert $S_{max}$ eingestellt, um die Detektion des nächsten natürlichen Herzschlages zu ermöglichen. Erst wenn diese Detektion erfolgt ist, wird für den darauffolgenden mittels der zweiten Detektoreinrichtung (43) detektierten natürlichen Herzschlag überprüft, ob eine Detektion auch mittels der ersten Detektoreinrichtung (27) mit der um einen weiteren Schritt E erhöhten Empfindlichkeit S erfolgt. Dies wiederholt sich solange, bis eine neue Mindest-Empfindlichkeit T gefunden ist, mit der die erste Detektoreinrichtung (27) einen mittels der zweiten Detektoreinrichtung (43) detektierten natürlichen Herzschlag ebenfalls detektiert. Daraufhin wird die neue Mindest-Empfindlichkeit T und die neue Empfindlichkeit S wie unmittelbar vorstehend beschrieben gesetzt und mit diesen Werten in den "NORMAL MODE" übergegangen, wobei dann das Sicherheits-Marginal X und das zusätzliche Sicherheits-Marginal Y vorhanden sind.

Wird ein mittels der zweiten Detektoreinrichtung (43) detektierter natürlicher Herzschlag mit der ersten Detektoreinrichtung (27) selbst dann nicht detektiert, wenn diese auf ihre maximale Empfindlichkeit $S_{max}$ eingestellt ist, wird für den weiteren Betrieb des Herzschrittmachers die Empfindlichkeit S der ersten Detektoreinrichtung (27) auf ihren maximalen Wert $S_{max}$ eingestellt. Ausserdem werden Massnahmen getroffen, die sicherstellen, dass bei der nächsten Kommunikation des Programmers (37) mit dem Herzschrittmacher (1) der behandelnde Arzt darauf hingewiesen wird, dass die Empfindlichkeit der ersten Detektoreinrichtung (27) auf ihren maximalen Wert $S_{max}$ eingestellt wurde. Anstelle der Einstellung der Empfindlichkeit der ersten Detektoreinrichtung auf ihren maximalen Wert $S_{max}$ kann auch vorgesehen sein, dass die erste Detektoreinrichtung (27) deaktiviert wird und die zweite Detektoreinrichtung (43) an ihre Stelle tritt.

Detektiert die erste Detektoreinrichtung (27), während ihre Empfindlichkeit S auf den Wert S = T-X eingestellt ist, zu Anfang des beschriebenen Betriebsverfahrens einen der vier mittels der zweiten Detektoreinrichtung (43) detektierten natürlichen Herzschläge nicht, bedeutet dies, dass die Mindest-Empfindlichkeit T der ersten Detektoreinrichtung (27) um mehr als das Sicherheits-Marginal X erhöht werden muss, um überhaupt eine Detektion eines mittels der zweiten Detektoreinrichtung (43) detektierten natürlichen Herzschlages mit der ersten Detektoreinrichtung (27) zu ermöglichen. In diesem Falle wird daher zunächst eine neue Mindest-Empfindlichkeit T festgesetzt, die der zuvor eingestellten Empfindlichkeit S entspricht. Im Anschluss daran wird zur Ermittlung der Mindest-Empfindlichkeit T in der zuvor beschriebenen Weise verfahren, worauf nach Einstellung einer entsprechenden Empfindlichkeit S die Rückkehr in den "NORMAL MODE" erfolgt.

Zwischen zwei aufeinanderfolgenden ersten Zeitpunkten wird im Falle der selbsttätigen Einstellung der Empfindlichkeit S der ersten Detektoreinrichtung (27) ähnlich wie im Falle der selbsttätigen Einstellung des Energiegehaltes A der Stimulationsimpulse zu wenigstens einem zweiten Zeitpunkt, sind mehrere zweite Zeitpunkte vorgesehen, sind diese durch Zeitintervalle in der Grössenordnung von Minuten getrennnt, geprüft, ob die im "NORMAL MODE" vorhandene Einstellung der Empfindlichkeit S im wesentlichen noch korrekt ist. Zu diesem Zweck wird der "NORMAL MODE" verlassen und die zweite Detektoreinrichtung (43) aktiviert. Es wird dann für eine definierte Anzahl von mittels der zweiten Detektoreinrichtung (43) detektierten natürlichen Herzschlägen, im Falle der Fig. 5 sind dies vier natürliche Herzschläge, geprüft, ob diese Herzschläge auch mittels der ersten Detektoreinrichtung (27) detektiert werden. Ist dies für alle natürlichen Herzschläge der definierten Anzahl der Fall, erfolgt die Rückkehr in den "NORMAL MODE". Bleibt für einen der natürlichen Herzschläge die Detektion mittels der ersten Detektoreinrichtung (27) aus, wird zunächst die Mindest-Empfindlichkeit T neu festgesetzt, und zwar auf einen Wert, der der zuvor in "NORMAL MODE" vorhandenen Empfindlichkeit S entspricht. Im Anschluss daran erfolgt die Ermittlung einer neuen Mindest-Empfindlichkeit T in der zuvor schon beschriebenen Weise, und nach der Festsetzung und Einstellung einer neuen Empfindlichkeit S für die erste Detektoreinrichtung (27) die Rückkehr in den "NORMAL MODE".

Die ersten und zweiten Zeitpunkte werden zweckmässigerweise ermittelt, indem der Mikroprozessor (5) eine entsprechend Anzahl von mittels des Quarzes (14) erzeugten Taktimpulsen abzählt.

Wesentliche Funktionen des Herzschrittmachers gemäss dem beschriebenen Ausführungsbeispiel sind durch einen geeignet programmierten Mikroprozessor (5) gesteuert. Die entsprechenden Funktionen können jedoch ohne weiteres auch durch eine herkömmlich aufgebaute Steuerlogik realisiert werden.

Sofern eine selbsttätige Einstellung der Empfindlichkeit der zur Detektion natürlicher Herzschläge dienenden Detektoreinrichtung nicht gewünscht wird, kann der gesamte Kanal 15 einschliesslich der ersten Detektoreinrichtung (27) entfallen. Deren Funktion übernimmt dann die (zweite) Detektoreinrichtung (43). Anders als im Falle des beschriebenen Ausführungsbeispiels erfolgt dann die Detektion natürlicher Herzschläge ausschliesslich mittels der Detektoreinrichtung (43). Demzufolge erfolgt die Inhibierung der Abgabe von Stimulationsimpulsen anhand der Ausgangssignale der Detektoreinrichtung (43), so wie dies im Zusammenhang mit der Detektoreinrichtung (27) beschrieben ist.

Obwohl die Erfindung ausschliesslich anhand eines Herzschrittmachers erläutert ist, kann sie auch bei anderen medizinischen Geräten Verwendung finden, die eine Detektoreinrichtung mit einstellbarer Empfindlichkeit aufweisen.

## Patentansprüche

1. In den Körper eines Lebewesens implantierbares medizinisches Gerät mit Mitteln (20) zum elektrischen Stimulieren von Gewebekontraktionen mit einstellbarer Stimulationsintensität (A), mit einer Detektoreinrichtung (43) zur Detektion stimulierter Gewebekontraktionen und mit Stellmitteln (5, 24) zum Einstellen der Stimulationsintensität (A), die die Stimulationsintensität (A) selbsttätig derart einstellen, dass die Detektoreinrichtung (43) nach einer Stimulation eine stimulierte Gewebekontraktion detektiert, **dadurch gekennzeichnet,** dass die Detektoreinrichtung (43) stimulierte Gewebekontraktionen in einem der elektrischen Impedanz des stimulierten Gewebes entsprechenden Signal (IS) detektiert.

2. Gerät nach Anspruch 1, **dadurch gekennzeichnet,** dass die Detektoreinrichtung (43) eine modulierbare Stromquelle (52) eine Demodulatorschaltung (56) eine mit der Stromquelle (52) und der Demodulatorschaltung (56) verbundene Oszillatorschaltung (53), die ein periodisches Oszillatorsignal (P) erzeugt, und eine Detektorschaltung (57, 58) aufweist, wobei das Oszillatorsignal (P) die Stromquelle (52) derart moduliert, dass diese einen Wechselstrom (Q) konstanter Amplitude abgibt, der mit dem Oszillatorsignal (P) synchronisiert ist, wobei die Stromquelle (52) zwei Anschlüsse aufweist, an die eine bipolare Elektrode (3) anschliessbar, wobei der Wechselspannungsanteil (R) der über der Stromquelle (52) abfallenden Spannung der Demodulatorschaltung (56) zugeführt ist, die mittels des Oszillatorsignals (P) mit der Stromquelle (52) synchronisiert ist und den Wechselspannungsanteil (R) demoduliert, und wobei das demodulierte Signal (U) der Detektorschaltung (57, 58) zugeführt ist.

3. Gerät nach Anspruch 1 oder 2, **dadurch gekennzeichnet**, dass die Stellmittel (5, 24) die Stimulationsintensität (A) auf einen Wert einstellen, der um ein Sicherheits-Marginal (M, N) der Stimulationsintensität (A) höher als ein Mindestwert (V) der Stimulationsintensität (A) ist, bei dem die Detektoreinrichtung (43) nach jeder Stimulation eine stimulierte Gewebekontraktion detektiert.

4. Gerät nach Anspruch 3, **dadurch gekennzeichnet**, dass die Stellmittel (5, 24) zur Ermittlung des Mindestwertes (V) die Stimulationsintensität (A) ausgehend von einem Wert, bei dem die Detektoreinrichtung (43) wenigstens nach einzelnen Stimulationen keine stimulierte Gewebekontraktion detektiert, allmählich gerade soweit erhöhen, bis die Detektoreinrichtung (43) nach jeder Stimulation eine stimulierte Gewebekontraktion detektiert, und dass die Stellmittel (5, 24) zur Ermittlung desjenigen Wertes der Stimulationsintensitäts (A), bei dem die Detektoreinrichtung (43) wenigstens nach einzelnen Stimulationen keine stimulierte Muskelkontraktion detektiert, die Stiumulationsintensität (A) ausgehend von einem Wert, bei dem die Detektoreinrichtung (43) nach jeder Stimulation eine stimulierte Gewebekontraktion detektiert, allmählich verringern.

5. Gerät nach einem der Ansprüche 1 bis 4, **dadurch gekennzeichnet**, dass die Mittel (20) zum Stimulieren Gewebekontraktionen nur beim Ausbleiben spontaner Gewebekontraktionen stimulieren und dass eine weitere Detektoreinrichtung (27) zur Detektion spontaner Gewebekontraktionen anhand eines der Kontraktionstätigkeit des zu stimulierenden Gewebes entsprechenden elektrischen Signals (ES) vorgesehen ist.

6. Gerät nach einem der Ansprüche 1 bis 5, **dadurch gekennzeichnet**, dass das Gerät als Herzschrittmacher ausgebildet ist, dessen Mittel (20) zum Stimulieren die Herztätigkeit mit elektrischen Impulsen stimulieren, dass die Stellmittel (5, 24) zur Einstellung der Stimulationsintensität den Energiegehalt (A) der Stimulationsimpulse einstellen, und dass als Gewebekontraktionen Herzschläge detektiert werden.

7. Betriebsverfahren des Gerätes nach einem der Ansprüche 1 bis 6, **dadurch gekennzeichnet**, dass zu aufeinanderfolgenden ersten Zeitpunkten die Stimulationsintensität (A) selbsttätig in Abhängigkeit von dem Ergebnis der Detektion der stimulierten Gewebekontraktionen in dem der elektrischen Impedanz des stimulierten Gewebes entsprechenden Signal eingestellt wird.

8. Verfahren nach Anspruch 7, **dadurch gekennzeichnet**, dass zu wenigstens einem zwischen zwei aufeinanderfolgenden ersten Zeitpunkten liegenden zweiten Zeitpunkt selbsttätig geprüft wird, ob nach einer Stimulation mit der zuletzt eingestellten Stimulationsintensität (A = V + M bzw. A = V + M + N) eine stimulierte Gewebekontraktion detektiert wird, und dass, falls dies nicht der Fall ist, die Stimulationsintensität (A) selbsttätig eingestellt wird.

9. Verfahren nach Anspruch 7 oder 8, **dadurch gekennzeichnet**, dass bei der selbsttätigen Einstellung der Stimulationsintensität (A) der Mindestwert (V) der Stimulationsintensität (A) ermittelt wird.

10. Verfahren nach Anspruch 9, **dadurch gekennzeichnet**, dass als Stimulationsintensität (A) eine Stimulationsintensität (A = V + M) eingestellt wird, die um ein Sicherheits-Marginal (M) höher als der Mindestwert (V) der Stimulationsintensität (A) ist.

11. Verfahren nach Anspruch 10, **dadurch gekennzeichnet**, dass, falls ein Mindestwert (V) der Stimulationsintensität (A) ermittelt wird, der höher als der zuletzt ermittelte Mindestwert (V) ist, eine Stimulationsintensität (A = V + M + N) eingestellt wird, die um das Sicherheits-Marginal (M) und ein zusätzliches Sicherheits-Marginal (N) höher als der Mindestwert (V) der Stimulationsintensität (A) ist.

12. Verfahren nach einem der Ansprüche 7 bis 11, **dadurch gekennzeichnet**, dass, falls ein Mindestwert (V) der Stimulationsintensität (A) ermittelt wird, der höchstens gleich dem zuletzt ermittelten Mindestwert (V) ist, bei der anschliessenden Einstellung der Stimulationsintensität (A) ein zuvor gegebenenfalls vorhandenes zusätzliches Sicherheits-Marginal (N) der Stimulationsintensität (A) entfällt.

13. Verfahren nach einem der Ansprüche 9 bis 11, **dadurch gekennzeichnet**, dass zur Ermittlung des Mindestwertes (V) der Stimulationsintensität (A) geprüft wird, ob nach einer Stimulation mit auf den zuletzt ermittelten Mindestwert (V) eingestellter Stimulationsintensität (A = V) eine stimulierte Gewebekontraktion detektiert wird, und dass, wenn dies nicht der Fall ist, die Stimulationsintensität (A) solange jeweils um einen definierten Schritt (I) erhöht wird, bis nach einer Stimulation mit der auf den erhöhten Mindestwert (V) eingestellten Stimulationsintensität (A) eine stimulierte Gewebekontraktion detektiert wird, worauf der Einstellung der Stimulationsintensität (A) der erhöhte Mindestwert (V) zugrunde gelegt wird.

14. Verfahren nach einem der Ansprüche 9 bis 13, **dadurch gekennzeichnet**, dass zur Ermittlung des Mindestwertes (V) der Stimulationsintensität (A) geprüft wird, ob nach einer Stimulation mit auf den zuletzt ermittelten Mindestwert (V) eingestellter Stimulationsintensität (A = V) eine stimulierte Gewebekontraktion detektiert wird, und dass, wenn dies der Fall ist, die Stimulationsintensität (A) um einen definierten Schritt (I) verringert wird, wobei für den Fall, dass nach einer Stimulations mit der auf den verringerten Mindestwert (V = V-I) eingestellten Stimulationsintensität (A) eine stimulierte Gewebekontraktion detektiert wird, der verringerte Mindestwert (V = V-I) der Einstellung der Stimulationsintensität (A) als Mindestwert (V) zugrunde gelegt wird, während andernfalls der zuletzt ermittelte Mindestwert (V) beibehalten und der Einstellung der Stimulationsintensität (A) zugrunde gelegt wird.

15. Verfahren nach einem der Ansprüche 9 bis 13, **dadurch gekennzeichnet**, dass bei der selbsttätigen Einstellung der Stimulationsintensität (A) vor der Ermittlung des Mindestwertes (V) der Stimulationsintensität (A) geprüft wird, ob nach einer Stimulation mit der zuletzt eingestellten Stimulationsintensität (A = V + M bzw. A = V + M + N), verringert um das gegebenenfalls vorhandene zusätzliche Sicherheits-Marginal (N), eine stimulierte Gewebekontraktion detektiert wird, und dass, wenn dies nicht der Fall ist, bei der selbsttätigen Einstellung der Stimulationsintensität (A) als zuletzt ermittelter Mindestwert (V) der Stimulationsintensität (A) die zuletzt eingestellte Empfindlichkeit (A = V + M bzw. A = V + M + N), verringert um das gegebenenfalls vorhandene zusätzliche Sicherheits-Marginal (M), zugrunde gelegt wird.

16. Verfahren nach einem der Ansprüche 9 bis 13, **dadurch gekennzeichnet**, dass, falls zu spontane Gewebekontraktion detektiert wird, bei der selbsttätigen Einstellung der Stimulationsintensität (A) als zuletzt ermittelte Mindestwert (V) der Stimulationsintensität (A) der zuletzt eingestellte Wert der Stimulationsintensität (A = V + M bzw. A = V + M + N) zugrunde gelegt wird.

17. Verfahren nach einem der Ansprüche 8, 13 oder 16, **dadurch gekennzeichnet**, dass für den Fall, dass nach einer Stimulation keine stimulierte Gewebekontraktion detektiert wird, die Stimulationsintensität (A) für die nächste Stimulation auf ihren Maximalwert ($A_{max}$) eingestellt wird.

## Claims

1. Medical device which can be implanted into the body of a living organism, having means (20) for the electrical stimulation of tissue contractions with adjustable stimulation intensity (A), having a detector device (43) for the detection of stimulated tissue contractions and having regulating means (5, 24) for the setting of the stimulation intensity (A), which automatically set the stimulation intensity (A) in such a manner that the detector device (43) detects a stimulated tissue contraction after a stimulation, characterised in that the detector device (43) detects stimulated tissue contractions in a signal (IS) corresponding to the electrical impedance of the stimulated tissue.

2. Device according to Claim 1, characterised in that the detector device (43) exhibits a modulable current source (52), a demodulator circuit (56), an oscillator circuit (53), which is connected to the current source (52) and the demodulator circuit (56) and which generates a periodic oscillator signal (P), and a detector circuit (57, 58), the oscillator signal (P) modulating the current source (52) in such a manner that the latter gives off an alternating current (Q) of constant amplitude, which alternating current is synchronised with the oscillator signal (P), the current source (52) exhibiting two connections, to which a bipolar electrode (3) is connectable, the alternating voltage component (R) of the voltage dropping over the current source (52) being fed to the demodulator circuit (56), which is synchronised with the current source (52) by means of the oscillator signal (P) and demodulating the alternating voltage component (R), and the demodulated signal (U) being fed to the detector circuit (57, 58).

3. Device according to Claim 1 or 2, characterised in that the regulating means (5, 24) set the stimulation intensity (A) to a value which is greater, by a safety margin (M, N) of the stimulation intensity (A), than a minimum value (V) of the stimulation intensity (A), in which device the detector device (43) detects a stimulated tissue contraction after each stimulation.

4. Device according to Claim 3, characterised in that to determine the minimum value (V) the regulating means (5, 24) gradually increase the stimulation intensity (A), starting from a value at which the detector device (43) detects no stimulated tissue contraction at least after individual stimulations, just to such an extent that the detector device (43) detects a stimulated tissue contraction after each stimulation, and in that to determine that value of the stimulation intensity (A) at which the detector device (43) detects no stimulated muscle contraction at least after individual stimulations, the regulating means (5, 24) gradually reduce the stimulation intensity (A), starting from a value at which the detector device (43) detects a stimulated tissue contraction after each stimulation.

5. Device according to one of Claims 1 to 4, characterised in that the stimulating means (20) stimulate tissue contractions only in the absence of spontaneous tissue contractions, and in that a further detector device (27) for the detection of spontaneous tissue contractions by means of an electrical signal (ES) corresponding to the contraction activity of the tissue to be stimulated is provided.

6. Device according to one of Claims 1 to 5, characterised in that the device is designed as a cardiac pacemaker, the stimulating means (20) of which stimulate the cardiac activity by electrical pulses, in that to set the stimulation intensity the regulating means (5, 24) set the energy content (A) of the stimulation pulses, and in that heart beats are detected as tissue contractions.

7. Process for operating the device according to one of Claims 1 to 6, characterised in that at successive first instants the stimulation intensity (A) is automatically set as a function of the result of the detection of the stimulated tissue contractions in the signal corresponding to the electrical impedance of the stimulated tissue.

8. Process according to Claim 7, characterised in that at at least one second instant situated between two successive first instants an automatic check is made as to whether following a stimulation with the last set stimulation intensity (A = V + M or A = V + M + N) a stimulated tissue contraction is detected, and in that, if this is not the case, the stimulation intensity (A) is automatically set.

9. Process according to Claim 7 or 8, characterised in that upon the automatic setting of the stimulation intensity (A) the minimum value (V) of the stimulation intensity (A) is determined.

10. Process according to Claim 9, characterised in that as stimulation intensity (A) there is set a stimulation intensity (A = V + M), which is greater, by a safety margin (M), than the minimum value (V) of the stimulation intensity (A).

11. Process according to Claim 10, characterised in that, if a minimum value (V) of the stimulation intensity (A) is determined, which is greater than the last determined minimum value (V), a stimulation intensity (A = V + M + N) is set, which is greater, by the safety margin (M) and an additional safety margin (N), than the minimum value (V) of the stimulation intensity (A).

12. Process according to one of Claims 7 to 11, characterised in that, if a minimum value (V) of the stimulation intensity (A) is determined, which is at most equal to the last determined minimum value (V), in the course of the subsequent setting of the stimulation intensity (A) a possibly previously present additional safety margin (N) of the stimulation intensity (A) is dispensed with.

13. Process according to one of Claims 9 to 11, characterised in that to determine the minimum value (V) of the stimulation intensity (A) a check is made as to whether following a stimulation with stimulation intensity (A = V) set to the last determined minimum value (V) a stimulated tissue contraction is detected, and in that, if this is not the case, the stimulation intensity (A) is increased in each instance by a defined step (I), until such time as following a stimulation with the stimulation intensity (A) set to the increased minimum value (V) a stimulated tissue contraction is detected, whereupon the increased minimum value (V) is adopted as a basis for the setting of the stimulation intensity (A).

14. Process according to one of Claims 9 to 13, characterised in that to determine the minimum value (V) of the stimulation intensity (A) a check is made as to whether following a stimulation with stimulation intensity (A = V) set to the last determined minimum value (V) a stimulated tissue contraction is detected, and in that, if this is the case, the stimulation intensity (A) is reduced by a defined step (I), in which process, in the event that following a stimulation with the stimulation intensity (A) set to the reduced minimum value (V = V-I) a stimulated tissue contraction is detected, the reduced minimum value (V = V-I) is adopted as a basis as a minimum value (V) for the setting of the stimulation intensity (A), while otherwise the last determined minimum value (V) is retained and adopted as a basis for the setting of the stimulation intensity (A).

15. Process according to one of Claims 9 to 13, characterised in that in the automatic setting of the stimulation intensity (A) before the determination of the minimum value (V) of the stimulation intensity (A) a check is made as to whether following a stimulation with the last set stimulation intensity (A = V + M or A = V + M + N), reduced by the possibly present additional safety margin (N), a stimulated tissue contraction is detected, and in that, if this is not the case, in the automatic setting of the stimulation intensity (A) the last set sensitivity (A = V + M or A = V + M + N), reduced by the possibly present additional safety margin (M), is adopted as a basis as last determined minimum value (V) of the stimulation intensity (A).

16. Process according to one of Claims 9 to 13, characterised in that, in the event that at [lacuna] spontaneous tissue contraction is detected, in the automatic setting of the stimulation intensity (A) the last set value of the stimulation intensity (A = V + M or A = V + M + N) is adopted as a basis as last determined minimum value (V) of the stimulation intensity (A).

17. Process according to one of Claims 8, 13 or 16, characterised in that in the event that following a stimulation no stimulated tissue contraction is detected, the stimulation intensity (A) for the next stimulation is set to its maximum value ($A_{max}$).

**Revendications**

1. Appareil médical implantable dans le corps d'un être vivant et comportant des moyens (20) pour stimuler électriquement des contractions du tissu avec une intensité de stimulation réglable (A), et un dispositif de détection (43) servant à détecter les contractions stimulées du tissu et des moyens de réglage (5,24) servant à régler l'intensité de stimulation (A) et qui règlent automatiquement cette intensité grâce au fait que le dispositif de détection (43) détecte, après une stimulation, une contraction stimulée du tissu, caractérisé par le fait que le dispositif de détection (43) détecte des contractions stimulées du tissu dans un signal (IS) qui correspond à l'impédance électrique du tissu stimulé.

2. Appareil suivant la revendication 1, caractérisé par le fait que le dispositif de détection (43) comporte une source de courant modulable (52), un circuit démodulateur (56), un circuit formant oscillateur (53) raccordé à la source de courant (52) et au circuit démodulateur (56) et qui produit un signal périodique (P), un circuit de détection (57,58), le signal (P) de l'oscillateur modulant la source de courant (52) de telle sorte que cette source délivre un courant alternatif (Q) d'amplitude constante, qui est synchronisé avec le signal (P) de l'oscillateur, la source de courant (52) possédant deux bornes, auxquelles une électrode bipolaire (3) peut être raccordée, tandis que la composante de tension alternative (R) de la tension, qui chute dans la source de courant (52), est envoyée au circuit démodulateur (56), qui est synchronisé sur la source de courant (52) au moyen du signal (P) de l'oscillateur et démodule la composante de tension alternative (R), et le signal démodulé (U) est envoyé au circuit de détection (57,58).

3. Appareil suivant la revendication 1 ou 2, caractérisé par le fait que les moyens de réglage (5,24) règlent l'intensité de stimulation (A) à une valeur qui est supérieure, d'une marge de sécurité (M,N) de l'intensité de stimulation (A), à une valeur minimale (B) de l'intensité de stimulation (A), pour laquelle le dispositif de détection (43) détecte, après chaque stimulation, une contraction stimulée du tissu.

4. Appareil suivant la revendication 3, caractérisé par le fait que les moyens de réglage (5,24) servant à déterminer la valeur minimale (V) augmentent progressivement l'intensité de stimulation (A) à partir d'une valeur pour laquelle le dispositif de détection (43) ne détecte aucune contraction stimulée du tissu, au moins après quelques stimulations, précisément au point que le dispositif de détection (43) détecte, après chaque stimulation, une contraction stimulée du tissu, et que les moyens de réglage (5,24) servant à déterminer la valeur de l'intensité de stimulation (A), pour laquelle le dispositif de détection (43) ne détecte aucune contraction musculaire stimulée, au moins après quelques stimulations, réduisent progressivement l'intensité de stimulation (A) à partir d'une valeur, pour laquelle le dispositif de détection (43) détecte, après chaque stimulation, une contraction stimulée du tissu.

5. Appareil suivant l'une des revendications 1 à 4, caractérisé par le fait que les moyens (20) servant à stimuler des contractions du tissu réalisent une stimulation uniquement en l'absence de contractions spontanées du tissu et qu'il est prévu un autre dispositif de sélection (27) servant à détecter des contractions spontanées du tissu sur la base d'un signal électrique (ES) qui correspond à l'activité de contraction du tissu devant être stimulé.

6. Appareil suivant l'une des revendications 1 à 5, caractérisé par le fait que l'appareil est agencé sous la forme d'un stimulateur cardiaque, dont les moyens de stimulation (20) stimulent l'activité cardiaque au moyen d'impulsions électriques, que les moyens de réglage (5,24) servant à régler l'intensité de stimulation règlent le contenu en énergie (A) des impulsions de stimulation et que des battements cardiaques sont détectés en tant que contractions du tissu.

7. Procédé de fonctionnement d'un appareil suivant l'une des revendications 1 à 6, caractérisé par le fait que l'intensité de stimulation (A) est réglée automatiquement à des premiers instants successifs, en fonction du résultat de la détection des contractions de stimulation du tissu dans le signal qui correspond à l'impédance électrique du tissu stimulé.

8. Procédé suivant la revendication 7, caractérisé par le fait qu'à au moins un second instant se situant entre deux premiers instants successifs, un contrôle automatique est exécuté pour déterminer, si après une stimulation avec l'intensité de stimulation réglée en dernier lieu (A = V + M ou A = V + M + N), une contraction stimulée du tissu est détectée et que, si ce n'est pas le cas, l'intensité de stimulation (A) est réglée automatiquement.

9. Procédé suivant la revendication 7 ou 8, caractérisé par le fait que dans le cas du réglage automatique de l'intensité de stimulation (A), la valeur minimale (V) de l'intensité de stimulation (A) est déterminée.

10. Procédé suivant la revendication 9, caractérisé par le fait qu'une intensité de stimulation (A = V + M), qui est supérieure, d'une marge de sécurité (M), à la valeur minimale (V) de l'intensité de stimulation (A), est réglée en tant qu'intensité de stimulation (A).

11. Procédé suivant la revendication 10, caractérisé par le fait que, dans le cas de la détermination d'une valeur minimale (V) de l'intensité de stimulation (A), qui est supérieure à la valeur minimale (V) déterminée en dernier lieu, une intensité de stimulation (A = V + M + N) est réglée, intensité qui est supérieure, de la marge de sécurité (M) et d'une marge de sécurité supplémentaire (N), à la valeur minimale (V) de l'intensité de stimulation (A).

12. Procédé suivant l'une des revendications 7 à 11, caractérisé par le fait que dans le cas de la détermination d'une valeur minimale (V) de l'intensité de stimulation (A), qui est égale au maximum à la valeur minimale (V) déterminée en dernier lieu, lors du réglage ultérieur de l'intensité de stimulation (A), une marge supplémentaire de sécurité (N), éventuellement présente auparavant, de l'intensité de stimulation (A) est supprimée.

13. Procédé suivant l'une des revendications 9 à 11, caractérisé par le fait que pour la détermination de la valeur minimale (V) de l'intensité de stimulation (A), un contrôle est exécuté pour déterminer si, après une stimulation avec une intensité de stimulation (A = V) réglée sur la valeur minimale (V) déterminée en dernier lieu, une contraction stimulée du tissu est détectée, et que, lorsque ce n'est pas le cas, l'intensité de stimulation (A) est accrue respectivement d'un échelon défini (I) jusqu'à ce que, après une stimulation avec l'intensité de stimulation (A) réglée sur la valeur minimale (V) accrue, une contraction stimulée du tissu soit détectée, à la suite de quoi le réglage de l'intensité de stimulation (A) s'effectue sur la base de la valeur minimale (V) accrue.

14. Procédé suivant l'une des revendications 9 à 13, caractérisé par le fait que pour la détermination de la valeur minimale (V) de l'intensité de stimulation (A), une vérification est faite pour savoir si après une stimulation avec une intensité de stimulation (A = V) réglée sur la valeur minimale (V) déterminée en dernier lieu, une contraction stimulée du tissu est détectée et que, lorsque c'est le cas, l'intensité de stimulation (A) est réduite d'un échelon défini (I), auquel cas, si, après une stimulation avec l'intensité de stimulation (A) réglée sur la valeur minimale réduite (V = V-I), une contraction stimulée du tissu est détectée, la valeur minimale réduite (V = V-I) est utilisée en tant que valeur minimale (V) pour le réglage de l'intensité de stimulation (A), alors que, sinon, la valeur minimale (V) déterminée en dernier lieu est conservée et est utilisée pour le réglage de l'intensité de stimulation (A).

15. Procédé suivant l'une des revendications 9 à 13, caractérisé par le fait que dans le cas du réglage automatique de l'intensité de stimulation (A) avant la détermination de la valeur minimale (V) de l'intensité de stimulation (A), une vérification est faite pour savoir si, après une stimulation avec l'intensité de stimulation réglée en dernier lieu (A = V + M ou A = V + M + N), diminuée de la marge de sécurité supplémentaire (N) éventuellement présente, une contraction stimulée du tissu est détectée et que, si ce n'est pas le cas, lors du réglage automatique de l'intensité de stimulation (A), on utilise comme valeur minimale (V) déterminée en dernier lieu de l'intensité de stimulation (A), la sensibilité réglée en dernier lieu (A = V + M ou A = V + M + N), diminuée de la marge de sécurité supplémentaire (M) éventuellement présente.

16. Procédé suivant l'une des revendications 9 à 13, caractérisé par le fait que dans le cas où une contraction spontanée du tissu est détectée, lors du réglage automatique de l'intensité de stimulation (A), on prend comme base, en tant que valeur minimale (V) déterminée en dernier lieu de l'intensité de stimulation (A), la valeur réglée en dernier lieu de l'intensité de stimulation (A = V + M ou A = V + M + N).

**17.** Procédé suivant l'une des revendications 8, 13 ou 16, caractérisé par le fait que dans le cas où après une stimulation, aucune contraction stimulée du tissu n'est détectée, l'intensité de stimulation (A) pour la stimulation suivante est réglée sur sa valeur maximale ($A_{MAX}$).

FIG 1

EP 0 399 063 B1

FIG 2

FIG 3

FIG 4

FIG 5